(19) **Europäisches Patentamt**

**European Patent Office**

**Office européen des brevets**

(11) **EP 0 995 992 B1**

(12) **EUROPÄISCHE PATENTSCHRIFT**

(45) Veröffentlichungstag und Bekanntmachung des Hinweises auf die Patenterteilung:
**14.09.2005   Patentblatt 2005/37**

(51) Int Cl.$^{7}$: **G01N 33/52**

(21) Anmeldenummer: **99120058.5**

(22) Anmeldetag: **19.10.1999**

(54) **Spreitschichten, Netzmittel zu ihrer Herstellung und deren Verwendung in Teststreifen**

Spreading layers, wetting agents for their preparation and their use in test strips

Couches de diffusion, agents mouillants pour leurs préparation et leurs utilisation dans les bandes d'essai

(84) Benannte Vertragsstaaten:
**AT BE CH CY DE DK ES FI FR GB GR IE IT LI LU MC NL PT SE**

(30) Priorität: **23.10.1998   DE 19849008**

(43) Veröffentlichungstag der Anmeldung:
**26.04.2000   Patentblatt 2000/17**

(73) Patentinhaber: **Roche Diagnostics GmbH**
**68305 Mannheim (DE)**

(72) Erfinder: **Knappe, Wolfgang, Dr.**
**67061 Ludwigshafen (DE)**

(56) Entgegenhaltungen:
**EP-A- 0 109 012          EP-A- 0 821 233**
**WO-A-97/18036          DE-A- 2 729 283**
**DE-A- 2 752 352          DE-A- 3 042 857**
**US-A- 4 370 412          US-A- 5 695 947**
**US-A- 5 779 867**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft Spreitschichten umfassend ein mit Natrium-N-oleoyl-sarcosinat imprägniertes poröses Flächengebilde, dieHerstellung dieses Spreitmaterials unter Verwendung des Natrium-N-oleoyl-sarcosinats sowie Teststreifen, die das erfindungsgemäße Spreitmaterial enthalten.

Zur qualitativen oder quantitativen analytischen Bestimmung von Bestandteilen von Körperflüssigkeiten, insbesondere von Blut, werden oft sogenannte trägergebundene Tests verwendet. Bei diesen liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und darauf angebrachten Nachweisschichten als Testfelder bestehen. Es sind jedoch auch Testträger bekannt, die als quadratische oder rechteckige Plättchen gestaltet sind. In der folgenden Beschreibung soll die Bezeichnung "Teststreifen" auch Testträger umfassen, die nicht die Streifenform aufweisen.

Testträger der eingangs bezeichneten Art sind beispielsweise aus der deutschen Patentschrift 21 18 455 bekannt. Dort werden diagnostische Testträger zum Nachweis von Analyten in Flüssigkeiten beschrieben, die aus einer Tragschicht und mindestens einer die Nachweisreagenzien enthaltenden Nachweisschicht, deren nicht auf der Tragschicht anliegende Oberfläche mit einer Deckschicht versehen ist, bestehen. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu vermeiden. Zum Nachweis eines Analyts in einer Flüssigkeit wird ein solcher diagnostischer Testträger in eine entsprechende Flüssigkeit, vorzugsweise Urin, eingetaucht. Die Nachweisschicht kommt so mit einem sehr großen Überschuß an Flüssigkeit in Kontakt, der nicht von dem Testträger aufgenommen werden kann. Je nach der Dauer des Kontaktes der Nachweisschicht mit der zu untersuchenden Flüssigkeit können jedoch unterschiedliche Farbintensitäten beobachtet werden.

In der Regel werden um so positivere Resultate erhalten, je länger die Kontaktzeit ist. Bei einem großen Probenüberschuß ist daher eine korrekte quantitative Analytbestimmung auf diese Weise nicht möglich.

Andererseits ist ein für eine Testträgerkonstruktion zu geringes Probenvolumen eine häufige Ursache für falsche Meßwerte beim Diabetes-Monitoring, das heißt, der regelmäßigen Kontrolle des Blutes Diabeteskranker auf den Gehalt an Glucose.

Testträger mit möglichst geringem Volumenbedarf sind deshalb das Ziel vielfältiger derzeitiger Entwicklungen.

Aus der DE-A-3042857 sind Teststreifen bekannt, die auf ihren mehrschichtigen Analyseelementen eine Probenverteilungsschicht (Spreitschicht) aufweisen, die die Aufgabe hat, punktförmig aufgetragene Probeflüssigkeit gleichmäßig über das ganze Testelement zu verteilen. Diese Spreitschicht besteht aus einem Tuch oder einer Schaumstoffschicht, die durch Imprägnierung mit einem Netzmittel hydrophiliert und entweder auf die noch feuchte oberste Gelatineschicht des Analyseelements gepreßt oder mittels einer zusätzlichen Klebstoffschicht darauf fixiert wird.

In den Ausführungsbeispielen dieser Druckschrift wird als Spreitschicht ein Baumwollgewebe oder ein Filtertuch eingesetzt, zur Hydrophilierung dient das nichtionische Netzmittel Polyoxyethylen-nonylphenoxyether.

Diagnostische Testträger in Form von Teststreifen, die einen deutlichen Fortschritt in Bezug auf Reproduzierbarkeit der Testergebnisse selbst bei Applikation unterschiedlicher Probenvolumina und in Bezug auf hygienische Handhabbarkeit bieten, sind aus der EP-A-0 821 233 bekannt.

Sie enthalten eine Tragschicht mit einer darauf angeordneten, zur Bestimmung von Analyt in flüssiger Probe erforderliche Reagenzien enthaltenden Nachweisschicht und eine die Nachweisschicht überdeckende Auflage aus einem Netzwerk, die größer als die Nachweisschicht ist und die beiderseits der Nachweisschicht auf der Tragschicht, vorzugsweise über einen Abstandshalter, befestigt ist, auf der Nachweisschicht dagegen ohne Befestigung unmittelbar, d.h. diese ohne Abstand im wesentlichen vollflächig berührend, aufliegt. Das als Auflage eingesetzte Netzwerk soll hydrophil aber alleine nicht kapillaraktiv sein. Auch hier wird das Netzwerk zur Hydrophilierung mit einem Netzmittel, nämlich Natrium-dioctylsulfosuccinat, imprägniert.

Das die Nachweisschicht überdeckende Netzwerk besteht bei dieser Konstruktion vorzugsweise aus einem grobtitrigen, relativ grobmaschigen Monofilgewebe mit einer ausreichend großen Maschenweite, damit Flüssigkeit durch das Netz auf die Nachweisschicht gelangt (Seite 3, Zeile 12). Im Beispiel dieser Anmeldung wird ein Monofilgewebe mit einer Maschenweite von 280 μm eingesetzt. Ihm kommt eine wesentliche Funktion zu: Es leitet auf seine Oberfläche aufgebrachte Probenflüssigkeit schnell auf die darunterliegende Nachweisschicht weiter. Wenn die Nachweisschicht gesättigt ist, wird ein eventuell vorhandener Probenüberschuß in die über die Nachweisschicht hinausgehenden Randbereiche des Netzwerks abgeleitet. Auf diese Weise werden geringe Probenmengen der Nachweisschicht vollständig zur Verfügung gestellt, die längere Einwirkung eines Probenüberschusses, die zu Fehl-Positiv-Ergebnissen führen kann, aber vermieden.

In dieser Druckschrift ist auch eine Ausführungsform beschrieben, die zwei oder mehrere nebeneinander angeordnete Nachweisschichten aufweist, die zur Messung des gleichen oder verschiedener Analyte dienen sollen.

Die Verwendung von Testträgern in dieser Ausführungsform bietet natürlich auf den ersten Blick verlockende Vorteile bezüglich Arbeits- und Kostenersparnis, da es mit ihnen möglich sein sollte, mit einem einzigen Probenauftrag zwei oder gar mehrere Messungen auszuführen. In der Praxis ergeben sich aber beim Versuch, diese Ausführungsform einzusetzen, Schwierigkeiten, die darauf zurückzuführen sind, daß die Spreitwirkung des relativ grobmaschigen Netzwerks bei mehrfeldrigen Teststreifenkonstruktionen nicht ausreicht, die Analytproben auf beide Nachweisfelder zu verteilen. Nur bei sorgfältigstem Auftrag einer Analytprobe genau über der Grenze beider Testfelder - was in der Praxis nur selten zu erreichen ist - läßt sich eine Benetzung beider Testfelder erreichen, jedoch benötigt man dann zur vollständigen Benetzung der Testfelder ein Mindest-Probenvolumen von über 15 µl.

Auch der Versuch, eine Benetzung beider Testfelder dadurch zu erreichen, daß die Felder auf dem Träger ohne Zwischenraum unmitelbar nebeneinander plaziert werden, führt nicht zum Erfolg. Es zeigt sich, daß bereits der auch in diesem Fall immer noch vorhandene winzige Abstand der Testfelder von 5 bis 10 µm das Blut daran hindert, von einem auf das andere Feld "überzuspringen".

Im Stand der Technik zeigen sich somit zwar Ansätze zur weiteren Verbesserung und zur Ökonomisierung der Analyseverfahren unter Verwendung von Teststreifen, jedoch stellen sich deren Anwendung in der Praxis noch erhebliche Hindernisse in den Weg.

Es fehlt an einer technischen Lehre, Analytproben schnell und gleichmäßig über eine Testfeldanordnung zu verteilen, insbesondere wenn kleine Probevolumina zur Verfügung stehen und/oder gar mehrere Testfelder nebeneinander angeordnet sind. So besteht unter anderem das Bedürfnis, zum quantitativen Nachweis von Analyten, beispielsweise Glucose, in Kapillarblut bei einem Einfeldtest etwa 3 µl Blut in weniger als 2 Sekunden über die relativ große Fläche von 30 mm$^2$ gleichmäßig zu verteilen. Bei einem Zweifeldertest sollen gar ca. 5 µl Blut über zwei Testfelder von je ca. 30 mm$^2$ verteilt werden.

Auch der Zeitfaktor hat bei derartigen Verfahren eine große Bedeutung: Es geht keineswegs allein darum, die Testprozedur zu beschleunigen, sondern es kommt darauf an, den Analyt mit möglichst geringer Verzögerung in der gesamten Testschicht gleichmäßig zu verteilen, denn, wie oben bereits gesagt, kann sich ein Zeitversatz in einem Gradienten des Analysenergebnisses über das Testfeld niederschlagen, sodaß die Bestimmung mit einem erheblichen Unsicherheitsfaktor belastet wird.

Die aus diesen Forderungen resultierenden Aufgaben sind nach Kenntnis des Patentanmelders bisher noch nicht in zufriedenstellender Weise auf einfache Art gelöst worden.

Es wurde nun überraschenderweise gefunden, daß sich eine Reduzierung des für die Messung erforderlichen Probenvolumens auf ca. 4 µl erzielen und eine sehr gleichmäßige Verteilung der Probe sogar auf mehrere nebeneinander angeordnete Nachweisfelder in einer Zeit von unter 2 Sekunden erreichen läßt, wenn man die Nachweisfelder mit dem im Folgenden beschriebenen erfindungsgemäßen Spreitmaterial überdeckt.

Das erfindungsgemäße Spreitmaterial umfaßt ein mit einem Netzmittel imprägniertes poröses Flächengebilde, das dadurch gekennzeichnet ist, daß das Netzmittel Natrium-N-oleoyl-sarcosinat ist. Natrium-N-oleoyl-sarcosinat kann leicht durch Umsetzung von N-Oleoyl-sarcosin (z.B. Handelsprodukt Crodasinic® O der Fa. Croda, Nettetal) mit einer äquivalenten Menge Natronlauge hergestellt werden.

Im Zusammenhang mit der stofflichen Beschreibung des erfindungsgemäßen Spreitmaterials bedeutet das Merkmal "imprägniert", daß das Material auf seiner für Flüssigkeiten zugänglichen Oberfläche eine Auflage des Imprägnierungsmittels trägt, d.h. daß auch Poren und Filamentzwischenräume mit diesem Mittel "ausgekleidet" sind. Die Auflage der Verbindung der Formel I bewirkt eine besonders vorteilhafte Hydrophilierung der porösen Flächengebilde, die ihre gute Eignung als Spreitmaterial begründet.

Naturgemäß ist die örtliche Oberflächenkonzentration des Imprägnierungsmittels von der Zugänglichkeit des betreffenden Oberflächenelements abhängig. Dies ist jedoch für die Hydrophilierungswirkung der oberflächlichen Auflage von untergeordneter Bedeutung.

Zur Erzielung einer hydrophil wirksamen Auflage auf erfindungsgemäßen Spreitmaterialien ist in der Regel eine Menge von 0,01 bis 2,0 Gew.-%, vorzugsweise von 0,03 bis 0,5 Gew.-% von Natrium-N-oleoyl-sarcosinat, bezogen auf das Gewicht des Materials vor der Imprägnierung ausreichend.

Die überlegene Spreitwirkung des erfindungsgemäßen Spreitmaterials ist eng verknüpft mit der Anwesenheit von Natrium-N-oleoyl-sarcosinat in dem Material. Die Überlegenheit des erfindungsgemäß eingesetzten Netzmittels gegenüber dem im nächstvergleichbaren Stand der Technik, der EP-A-0 821 233, eingesetzten läßt sich durch einen einfachen Versuch demonstrieren:

Ein Vliesstoff (Viledon® FO 2451/121 der Fa. Freudenberg), wird in einer 0,5 gew.-%igen wässrigen Lösung von Natrium-dioctyl-sulfosuccinat (dem in der EP-A-0 821 233 benutzten Netzmittel) bis zur Sättigung imprägniert und an der Luft getrocknet. Auf ein ca. 3x3 cm großes, waagerecht eingespanntes Stück des so imprägnierten Vliesstoffs wird eine Blutprobe von 5 bis 10 µl aufgesetzt. Das Blut bleibt halbkugelförmig auf der Oberseite des Vliesstoffs stehen, die Unterseite wird nicht feucht.

Wird die gleiche Blutmenge auf ein Stück des mit Natrium-dioctyl-sulfosuccinat imprägnierten Vliesstoffs aufgegeben, der auf einem Reagenzfilm liegt, so steht der Blutstropfen ebenfalls auf der Oberseite des Vlieses, ohne daß eine Benetzung des Films und damit eine Reaktion erfolgt. Erst bei mechanischem Durchdrücken des Blutes an einer Stelle, z.B. durch Berühren des Vlieses mit der Pipettenspitze, gelangt Blut auf die Unterseite des Vlieses und Spreitung und Reaktion erfolgen.

Wird der beschriebene Versuch in genau gleicher Weise wiederholt mit dem einzigen Unterschied, daß anstelle der Natrium-dioctyl-sulfosuccinat-Lösung eine wässrige, 0,1 gew.-%ige Lösung von Natrium-N-oleoyl-sarcosinat zur Imprägnierung des Vliesstoffs eingesetzt wird, so durchdringt das aufgesetzte Blut das Vlies und hängt halbkugelförmig auf der Unterseite. Liegt das imprägnierte Vlies beim Blutauftrag auf einem Reagenzfilm, so gelangt das Blut spontan auf die Unterseite und Spreitung und Reaktion erfolgen.

Die demonstrierte Überlegenheit von Natrium-N-oleoyl-sarcosinat bei ihrer Verwendung als Spreitmittel ist sehr überraschend. Netzmittel sind in größerer Zahl und aus verschiedenen Gruppen chemischer Verbindungen bekannt.

Gemeinsam ist ihnen, daß sie einen hydrophilen und einen hydrophoben Molekülabschnitt aufweisen.

In "Ullmanns Encyclopedia of Technical Chemistry", Vol.5, werden Netzmittel mit nichtionischem, anionischem, kationischem und amphoterem Charakter beschrieben. Auf der Seite 778 werden aus der Gruppe der anionischen Netzmittel Bernsteinsäurederivate, insbesondere Sulfobernsteinsäure-dialkylester als Netzmittel mit überragender Wirksamkeit hervorgehoben, die eine außerordentlich breite technische Anwendung gefunden haben.

Auf Seite 751 dieses Standardwerkes werden als Netzmittel auch Derivate von Aminocarbonsäuren, u.a. der Aminoessigsäure - die auch ein Strukturelement der erfindungsgemäß einzusetzenden Verbindung ist - genannt und in die Gruppe der amphoteren Netzmittel eingeordnet. Auf den Seiten 795/796 wird dazu ausgeführt, daß diese nur eine geringe technische Bedeutung erlangt haben, insbesondere wegen der starken Abhängigkeit ihrer Eigenschaften vom pH-Wert.

Es war daher nicht vorherzusehen, daß Natrium-N-oleoyl-sarcosinat bei seiner Verwendung als Spreitmittel den als exzellente Netzmittel bekannten Sulfobemsteinsäureestem erheblich überlegen ist.

Das dem erfindungsgemäßen Spreitmaterial zugrundeliegende poröse Flächengebilde ist ein textiles Flächengebilde aus Monofilamenten oder entsprechenden Multifilamentgarnen, das selbst nicht kapillaraktiv aber flüssigkeitsdurchlässig ist, und dessen Konstruktion und Material und/oder Hydrophilierungsausrüstung so gewählt wird, daß es, auf einer Unterlage aufliegend, 10 µl Wasser auf eine Fläche von mehr als 300 mm$^2$ spreitet.

Textile Flächengebilde, die diese Forderung erfüllen, finden sich unter handelsüblichen Materialien. Die grobe Auswahl geeigneter Textilmaterialien kann grundsätzlich nach der Devise "möglichst dünn bei möglichst kleinem Flächengewicht" erfolgen. Zur Feinauswahl geeigneter Materialien kann der folgende Spreit-Test dienen:

10 mm breite und mindestens 100 mm lange Streifen des zu prüfenden, erforderlichenfalls hydrophilierten Textilmaterials werden auf die mattierte Seite einer Polycarbonatfolie aufgelegt. Dann trägt man in der Mitte des Streifens 10 µl Wasser punktförmig auf und beobachtet seine Ausbreitung. Bei einem geeigneten Material soll der Ausbreitungsprozeß innerhalb von 5 bis 10 Sekunden abgeschlossen sein, und die benetzte Fläche mindestens 30x10 mm betragen.

Mit diesem Test lassen sich beispielsweise die gravierenden Unterschiede zwischen dem gemäß EP-A-0 821 233 als Spreitmaterial einzusetzenden Polyestergewebe PE 280 HC und erfindungsgemäß einzusetzenden Textilmaterialien, z.B. Polyestergewebe PE 38 HC oder Viledon-Vliesstoff FO 2451/121 wie folgt demonstrieren:

10 mm breite Streifen der zu testenden Textilmaterialien werden mit der gleichen Auflage (ca. 0,25 Gew.-%, bezogen auf Materialgewicht vor dem Imprägnieren) Natrium-N-oleoyl-sarcosinat imprägniert und getrocknet. Dann werden die imprägnierten Textilstreifen auf die mattierte Seite einer Polycarbonatfolie aufgelegt. Trägt man nun 10 µl Wasser punktförmig auf, so spreitet das Wasser in 5 bis 10 Sekunden über die in der folgenden Tabelle angegebenen Flächen und kommt dann zum Stillstand:

| Gemäß | Material | Fläche |
|---|---|---|
| EP-A-0821233 | PE 280 HC | 8 x 10 mm |
| Erfindung | PE 38 HC | 40 x 10 mm |
| Erfindung | Viledon FO 2451/121 | 50 x 10 mm |

Als Textilmaterialien, die für die Spreitung geringer Blutmengen besonders geeignet sind, haben sich Gewebe, Gewirke oder Vliese mit einer Dicke von 20 bis 200 µm, vorzugsweise 30 bis 100 µm und/oder einem Porenvolumen von 30 bis 85, vorzugsweise von 40 bis 75 %. erwiesen. Das Flächengewicht dieser für die Spreitung geringer Probenvolumina besonders geeigneter Materialien beträgt 10 bis 200, vorzugsweise 10 bis 50 g/m$^2$.

Besonders bevorzugt sind in diesem Rahmen die Materialien mit Dicken unter 100 µm insbesondere unter 50 µm und Flächengewichten von unter 50, vorzugsweise unter 25 g/m$^2$.

Bevorzugte Flächengebilde mit den genannten Dimensionen sind Gewebe und Vliese. Von den Geweben sind solche bevorzugt, die eine Maschenweite unter 200 µm, vorzugsweise von 20 bis 150 µm, insbesondere von 20 bis 60 µm, aufweisen, schiebefest sind und aus Monofilamenten mit Titern im Bereich von 2 bis 20 dtex, vorzugsweise von 4 bis 15 dtex, in Leinwandbindung hergestellt sind. Die Gewebe können eine nach dem Weben aufgebrachte Schiebefestausrüstung aufweisen. Vorzugsweise wird die Verschiebefestigkeit der Gewebe jedoch dadurch erreicht, daß den Monofilamenten im Zuge der Gewebeherstellung eine wellenförmige Verformung aufgeprägt wird und/oder daß Kette- und Schußfilamente an den Kreuzungspunkten leicht miteinander verschmolzen werden, wodurch sie gegen Verschieben gesichert werden.

Erfindungsgemäß eingesetzte Vliese sind Wirrvliese, vorzugsweise gebondete Spinnvliese. Sie bestehen aus endlosen Monofilamenten, deren Titer im Bereich von 0,5 bis 2,5 dtex, vorzugsweise von 0,8 bis 2,0 dtex liegt. Das Bonden kann durch Behandlung der Vliese mit Bondierungsmitteln erfolgen; bevorzugt ist jedoch das "autogene" Bonden, bei dem die Filamente an ihren Kreuzungspunkten miteinander leicht verschmolzen werden. Die Poren derartiger Vliese weisen naturgemäß erhebliche Größenunterschiede auf Überraschenderweise sind Vliese mit den oben angegebenen Merkmalen trotz großer Streubreite der Porengröße für den erfindungsgemäßen Einsatz hervorragend geeignet.

Die geringe oder nicht vorhandene Kapillaraktivität des Flächengebildes zeigt sich darin, daß es eine Steighöhe von Wasser bei 25°C von unter 2 mm in 10 sec. aufweist.

Als Fasermaterialien für die erfindungsgemäß einzusetzenden Textilmaterialien kommen sowohl natürliche Fasern wie Zellulose oder Eiweisfasern, halbsynthetische Fasern wie Acetat- oder Viskoseseide oder vollsynthetische Fasern wie Polyester-, Polyamid-, Polyurethan-, Polyacrylnitril-, Polyethylen oder Polypropylenfasern oder Mischungen aus den genannten Fasertypen in Betracht.

Bevorzugte Zellulosefasern sind Baumwollefasern, bevorzugte Eiweisfasern sind Wolle und Naturseide. Geeignete Acetatfasern sind 2,5- oder Triacetatfasern je nach dem gewünschten Hydrophiliegrad. Bevorzugte Synthesefasern bestehen aus Polyethylenterephthalat, Polyamid-6, Polyamid-6,6 oder modifiziertem Polyacrylnitril.

Wegen der hohen Stabilität gegen Umgebungseinflüsse und der Möglichkeit, bei der Herstellung mechanische und chemische Eigenschaften bedarfsgerecht zu modifizieren, sind synthetische, insbesondere thermoplastische, Fasern gegenüber den natürlichen bevorzugt.

Handelsübliche textile Flächengebilde, die, insbesondere nach einer Hydrophilierung, erfindungsgemäß eingesetzt werden können, sind beispielsweise das monofile Polyestergewebe Type PE nn HC oder das monofile Nylongewebe Type NY nn HC oder HD der Firma ZBF Mesh+Technology, Rüschlikon, CH, wobei nn von 20 bis 150 variiert, insbesondere Polyestergewebe Type PE 38 HC oder das monofile Nylongewebe Type NY 41 HC. Sehr gut geeignet für den erfindungsgemäßen Einsatz sind auch die "Viledon® - Vliesstoffe FO 2451, insbesondere der Typ FO 2451/121, der Firma Freudenberg, Weinheim, BRD.

Der Einsatz des erfindungsgemäßen Spreitmaterials ist natürlich nicht auf die Verwendung beim Aufbau von Teststreifen beschränkt, obwohl ihnen dabei - insbesondere beim Aufbau von diagnostischen Teststreifen - eine besondere Bedeutung zukommt. Vielmehr kann das erfindungsgemäße Spreitmaterial in der Technik überall da eingesetzt werden, wo es auf die schnelle und gleichmäßige Verteilung von Flüssigkeiten über relativ große Flächen ankommt, wie z.B. bei der Entwicklung extrem großformatiger Silberhalogenidbilder mit beschränkter Entwicklermenge und bei bestimmten Tontrennungsverfahren in der künstlerischen Photographie.

Ein Gegenstand der vorliegenden Erfindung ist auch die Verwendung von Natrium-N-oleoyl-sarcosinat zur Herstellung eines Spreitmaterials.

Natrium-N-oleoyl-sarcosinat kann zur Herstellung des Spreitmaterials in reiner Form oder in Form von Zubereitungen, insbesondere in Form von Lösungen oder flüssigen Zubereitungen verwendet werden.

Eine Zubereitung kann aus einer Lösung oder einer feinteiligen Dispersion von Natrium-N-oleoyl-sarcosinat in Wasser, einem inerten organischen Lösungsmittel oder einem Lösungsmittel/Wasser-Gemisch bestehen. Sie kann auch eine Emulsion einer wäßrigen Lösung von Natrium-N-oleoyl-sarcosinat in einem mit Wasser nicht mischbaren Lösungsmittel sein.

Die Zubereitungen können neben Natrium-N-oleoyl-sarcosinat auch noch weitere Zusätze und/oder Hilfstoffe enthalten, die entweder synergistisch mit diesemzusammenwirken oder die zum Beispiel als Imprägnierungshilfsmittel, als Stabilisatoren oder als Schutzkolloide wirken. Weiterhin können die Zubereitungen neben Natrium-N-oleoyl-sarcosinat Stoffe enthalten, die dem Spreitmaterial Zusatzfunktionen oder andere vorteilhafte Eigenschaften verleihen. So können die Zubereitungen beispielsweise auch fein dispergierte anorganische oder organische Filtermaterialien oder Füller enthalten. Auch Farbstoffe, die dem Spreitmaterial eine Kennzeichnungsfarbe verleihen oder die sich zur Verbesserung der Erkennbarkeit von Farbreaktionen der Nachweisschichten oder der vollständigen Durchfeuchtung des Spreitmaterials eignen, oder die UV-Strahlung absorbieren, sind Beispiele für Zusatzstoffe, die in den Zubereitungen des erfindungsgemäß zu verwendenden Natrium-N-oleoyl-sarcosinats enthalten sein können.

Ein weiterer Gegenstand der Erfindung ist auch ein Verfahren zur Herstellung eines Spreitmaterials durch Imprägnierung eines porösen Flächengebildes mit einem Netzmittel oder einer Netzmittelzubereitung, gegebenenfalls Einstellung des imprägnierten Flächengebildes auf eine vorbestimmte Netzmittelaufnahme und gegebenenfalls Trocknen des Materials,

das dadurch gekennzeichnet ist, daß als Netzmittel Natrium-N-oleoyl-sarcosinat eingesetzt wird.

In dem erfindungsgemäßen Verfahren einzusetzende Netzmittelzubereitungen sind bereits oben beschrieben worden.

Für die Imprägnierung eignen sich alle Maßnahmen, die zu einer statistisch gleichmäßigen Verteilung des Netzmittels in dem porösen Flächengebilde geeignet sind. In der Regel wird das Netzmittel in Form einer Lösung oder einer flüssigen Zubereitung appliziert.

Die eingesetzten Imprägnierungsmittel enthalten zweckmäßigerweise 0,01 bis 2 Gew.-%, vorzugsweise 0,03 bis 1 Gew.-%, insbesondere 0,07 bis 0,3 Gew.-% Netzmittel.

Die Applikation kann in jeder üblichen Weise erfolgen, beispielsweise durch Tauchen, Aufsprühen, oder durch Bürstenauftrag. Es ist auch möglich, die porösen Materialien mit Feinstäuben von Natrium-N-oleoyl-sarcosinats oder fester Zubereitungen desselben einzupudern.

Vorzugsweise wird Natrium-N-oleoyl-sarcosinat in Form von wässrigen Lösungen appliziert.

Die applizierte Menge des Natrium-N-oleoyl-sarcosinats oder der entsprechenden Zubereitungen wird so bemessen, oder ein aufgebrachter Überschuß soweit entfernt, daß auf dem porösen Material eine Auflage von 0,01 bis 2,0 Gew.-%, vorzugsweise von 0,03 bis 0,5 Gew.-% des Natrium-N-oleoyl-sarcosinats bezogen auf das Gewicht des Materials vor der Imprägnierung, verbleibt.

Da das Netzmittel in der Regel in Form von Lösungen oder flüssigen Zubereitungen appliziert wird, ist nach dem Imprägnieren eine Trocknung des imprägnierten Materials erforderlich. Diese kann in jeder für Textilmaterialien oder offenporige Schaumstoffe geeigneten Weise erfolgen. Üblicherweise trocknet man das Material bei Temperaturen zwischen 10°C und dem Siedepunkt der im Applikationsmittel enthaltenen flüssigen Phase, vorzugsweise bei 20 bis 80°C. Der Trocknungsprozeß kann durch Vakuum und/oder Luftumwälzung unterstützt werden. Die Wärmezufuhr kann durch Konvektion mit einem Wärmeträger, durch Kontakt mit Heizelementen oder durch Strahlung erfolgen. Zweckmäßigerweise erfolgt die Trocknung der Flächengebilde in ausgebreitetem Zustand.

Neben dem erfindungsgemäßen Spreitmaterial ist auch ein Teststreifen Gegenstand der vorliegenden Erfindung, aus einem flexiblen flächenförmigen Träger, auf dem ein oder mehrere Testfelder nebeneinander angeordnet sind, die jeweils eine oder mehrere übereinanderliegende Nachweisschichten tragen, der dadurch gekennzeichnet ist, daß die Testfelder durch ein oben beschriebenes erfindungsgemäßes Spreitmaterial abgedeckt sind.

Vorzugsweise soll der Teststreifen diagnostischen Zwecken dienen.

Besonders vorteilhafte Teststreifen haben zwei unmittelbar aneinandergrenzende - wobei in der Praxis auch hierbei ein mikroskopischer Spalt von ca. 5-10 μm vorhanden ist -

oder durch einen Spalt getrennte ein- oder mehrschichtige Testfelder für den gleichen oder verschiedene Analyte. Es ist auch möglich, mehr als zwei Testfelder auf einem Teststreifen unterzubringen.

Die auf den Testfeldern angebrachten Nachweisschichten enthalten Reagenzien für den Nachweis eines diagnostisch verwertbaren Analyts. Der Nachweis des gleichen Analyts auf zwei getrennten Testfeldern kann von Interesse sein, wenn die Nachweisschichten eine qualitative, halbquantitative oder quantitative Beurteilung von sehr unterschiedlichen Konzentrationen des Analyts ermöglichen sollen, oder wenn einfach eine Beurteilung der Reproduzierbarkeit des Meßergebnisses erwünscht ist. Von besonderem Interesse ist natürlich der Fall, wo die beiden Nachweisschichten den gleichzeitigen qualitativen Nachweis oder eine halbquantitative oder quantitative Bestimmung von zwei verschiedenen, insbesondere diagnostisch interessanten, Analyten gestatten.

Das Spreitmaterial ist auf dem oder den Nachweisfeldern nicht fixiert sondern liegt nur lose auf. Im Gegensatz zu der aus der DE-A-30 42 857 (Seite 10) bekannten Lehre zur Herstellung von Teststreifen werden sie nicht in die Nachweisschichten eingepreßt oder mit ihnen verklebt. Eine derartige feste Verbindung macht die beschriebene Spreitfunktion unmöglich.

Die Befestigung des Spreitmaterials auf dem Teststreifen erfolgt lediglich beiderseits des Testfeldes bzw. der Testfelder, vorzugsweise auf mit Adhäsionsschichten versehenen Abstandshaltern. Bei dieser Art der Befestigung kann das Spreitmaterial allerdings in ungünstigen Fällen über dem Testbereich eine Falte bilden, wenn die in der Regel flexiblen Teststreifen bei der Verwendung einer Biegung unterworfen werden. Die Faltenbildung beeinträchtigt die Verteilung der Reagenzprobe über den Testbereich. Diese Faltenbildung läßt sich vermeiden, wenn die Auflage aus dem erfindungsgemäßen Spreitmaterial aus einem oder mehreren flächenförmigen Auflageelementen besteht, die auf dem flexiblen Teststreifen so befestigt sind, daß zumindest ein Teil ihrer Fläche gegenüber der von diesem Teil abgedeckten Fläche des Teststreifens in Richtung der beim Biegen des Streifens erzeugten Krümmung frei verschiebbar ist. Diese bevorzugte Art der Befestigung eines Auflageelements der erfindungsgemäßen Auflage auf dem flexiblen Teststreifen erfolgt durch mindestens einen, vorzugsweise mindestens zwei, Befestigungspunkte, die in einem zusammenhängenden Flächenbereich (Befestigungsbereich) des Auflageelements liegen, der sich zwischen den in Krümmungsrichtung liegenden Kanten des Auflageelements erstreckt und dessen Abgrenzung vom frei verschiebbaren Teil des Auflageelements im wesentlichen geradlinig und quer zur Krümmungsrichtung verläuft.

Für den Fall, daß der Teststreifen zwei Testfelder aufweist, ist deren Abdeckung durch eine erfindungsgemäße Spreitauflage der oben beschriebenen Art besonders praktisch, bei der die Testfelder durch zwei Auflageelemente abgedeckt werden, die so auf dem Teststreifen fixiert sind, daß ihre verschiebbaren Bereiche gegeneinander gerichtet sind und sich überlappen.

Dabei wird eine optimale Verteilung der Probe dann erreicht, wenn die Überlappung der beiden Auflageelemente über der Trennungslinie zwischen den beiden Testfeldern und vorzugsweise symmetrisch dazu liegt.

Gerade bei Teststreifen, deren einwandfreier Funktion eine besondere Bedeutung zukommt, die auch unter verschiedenen Einsatzbedingungen und bei der Anwendung durch mehr oder weniger geübte Laien gewährleistet sein muß, stellt die erfindungsgemäße Befestigungsart, durch die eine Faltenbildung bei Biegung des Teststreifens vermieden wird, einen sehr wertvollen Beitrag zur Anwendungssicherheit dar.

Vorzugsweise sind die Testfelder auf dem Testträger in Richtung seiner Längsachse hintereinander montiert und die Spreitauflage-Elemente, in der gleichen Richtung gesehen, in einem vor und einem hinter den Testfeldern gelegenen Flächenbereich auf dem flexiblen Träger fixiert.

Weiterhin ist es bevorzugt, daß die Spreitauflage-Elemente auf Abstandshaltern fixiert sind, die etwa die Dicke der Nachweisschichten haben.

Schließlich hat es sich als zweckmäßig erwiesen, wenn die Anordnung von Nachweisschichten und Auflagen auf dem Teststreifen mit einem inerten flächenförmigen Material so abgedeckt ist, daß nur im Bereich der Überlappung der Auflageelemente in Richtung der Längsachse des Teststreifens gesehen, eine für den Probenauftrag ausreichende Strecke freibleibt, die in der Regel 2 bis 5 mm beträgt.

Der Träger des Teststreifens besteht aus einem transparenten Material und/oder weist im Bereich der Testfelder Durchbrechungen gleicher oder unterschiedlicher Form auf, durch die die Unterseite der Nachweisschichten inspiziert werden können.

Vorteilhaft, insbesondere für eine Automatisierung der Testauswertung ist es, wenn der Träger des Teststreifens Justierungsmarkierungen in Form von zusätzlichen Bohrungen, Ausstanzungen oder Kerben aufweist.

Zur Veranschaulichung der obigen und der folgenden Ausführungen dienen die Figuren 1 bis 6.

Die Figur 1 zeigt eine perspektivische Aufsicht, die Figur 2 einen Schnitt längs der Schnittlinie A-A', die Figur 3 eine Aufsicht auf die Unterseite einer Ausführungsform eines erfindungsgemäßen Teststreifens.

Die Figur 4 zeigt eine perspektivische Aufsicht, die Figur 5 einen Schnitt längs der Schnittlinie A-A', die Figur 6 eine Aufsicht auf die Unterseite einer Ausführungsform eines erfindungsgemäßen Teststreifens bei der die erfindungsgemäße Spreitauflage aus zwei Auflageelementen besteht, die in bevorzugter Weise auf dem Teststreifen befestigt sind.

Die in den Figuren verwendeten Bezugszeichen haben folgende Bedeutung:

| | |
|---|---|
| 1: | Teststreifen |
| 2: | Flexibler Träger |
| 3 und 3a: | Nachweisschichten |
| 4 und 4a: | Abstandshalter |
| 5 und 5a: | Adhäsionsschichten |
| 6: | Erfindungsgemäße Auflage |
| 6 und 6a: | Erfindungsgemäße Auflageelemente |
| 7 und 7a: | Befestigungsbereich |
| 8 und 8a: | Verschiebbarer Bereich |
| 9 und 9a: | Schutzabdeckung |
| 10: | Auftragsbereich |
| 11 und 11a: | Markierung der Grenzen der Nachweisfelder |
| 12: | Markierung der Einschubrichtung |

13: Positionierungsbohrung

14 und 15: Beobachtungs- und Meßöffnungen

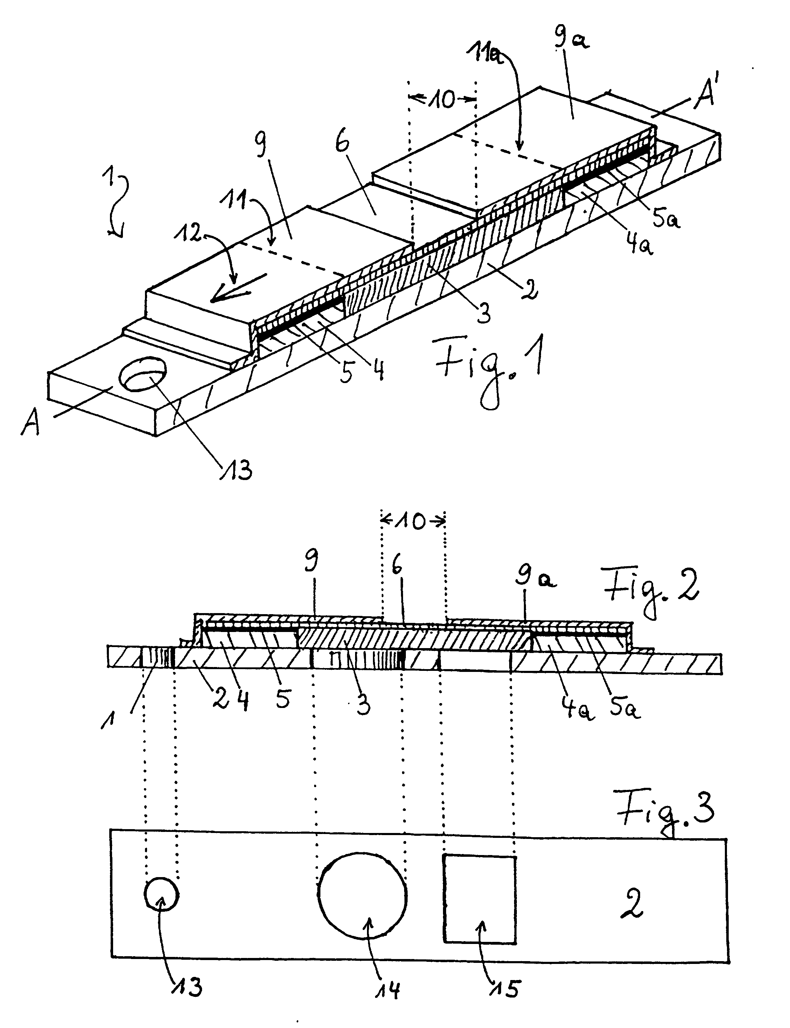

Die Figur 1 zeigt eine perspektivische Aufsicht, die Figur 2 einen Schnitt längs der Schnittlinie A-A'in Figur 1, die Figur 3 eine Aufsicht auf die Unterseite einer Ausführungsform eines erfindungsgemäßen Teststreifens mit einer Nachweisfläche und einer erfindungsgemäßen Spreitauflage. Diese Darstellung erfolgt ohne Maßstab um den Aufbau klar ersichtlich machen zu können. Eine konkrete Dimensionierung dieser Ausführungsform kann dem Ausführungsbeispiel 1 entnommen werden.

Der in Fig. 1 perspektivisch, in Fig. 2 im Schnitt und in Fig. 3 von unten gezeigte erfindungsgemäße diagnostische Teststreifen (1) weist auf einer Tragschicht (2) eine Nachweisschicht (3) auf, die durch die Spreitauflage (6) überdeckt ist. Neben der Nachweisschicht (3) ist die Spreitauflage (6) mittels Abstandhaltern (4,4a) und Adhäsionsschichten (5,5a) auf der Tragschicht (2) befestigt. Diese Abstandhalter können in der Praxis auch Schmelzkleberflächen oder zweiseitig klebende Bänder sein, die die Spreitauflage (6) auf der Tragschicht (2) fixieren. Idealerweise besitzen die Abstandhalter mit ihren Klebeflächen in etwa die gleiche Dicke wie die Nachweisschicht (3). Der hier dargestellte Aufbau weist ferner Abdeckungen (9,9a) auf, die auf der Tragschicht (2) und der Spreitauflage (6) befestigt sind. Sie sind so angeordnet, daß sie die über die Nachweisschicht (3) hinausragenden Bereiche und einen Teil der über der Nachweisschicht liegenden Fläche der Spreitauflage (6) überdecken. Sie lassen jedoch einen Bereich über der Mitte der Nachweisschicht frei,der die Probenauftragsstelle (10) darstellt. Hierauf wird die zu untersuchende Probenflüssigkeit aufgegeben. Die linke Abdeckung (9) enthält einen aufgedruckten Pfeil (12), der dem Anwender zeigt, mit welchem Ende der Testträger (1) in ein Meßgerät gelegt oder geschoben werden soll. Das Positionierloch (13) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (13) hineinragt und so den Testträger (1) an einer vorbestimmten Stelle festhält.

Die Figur 3 zeigt die Unterseite des erfindungsgemäßen Teststreifens mit der im Träger (2) angebrachten Positionierungsbohrung (13) und der runden Beobachtungsöffnung (14), durch die die Nachweisschicht inspiziert und vermessen werden kann.

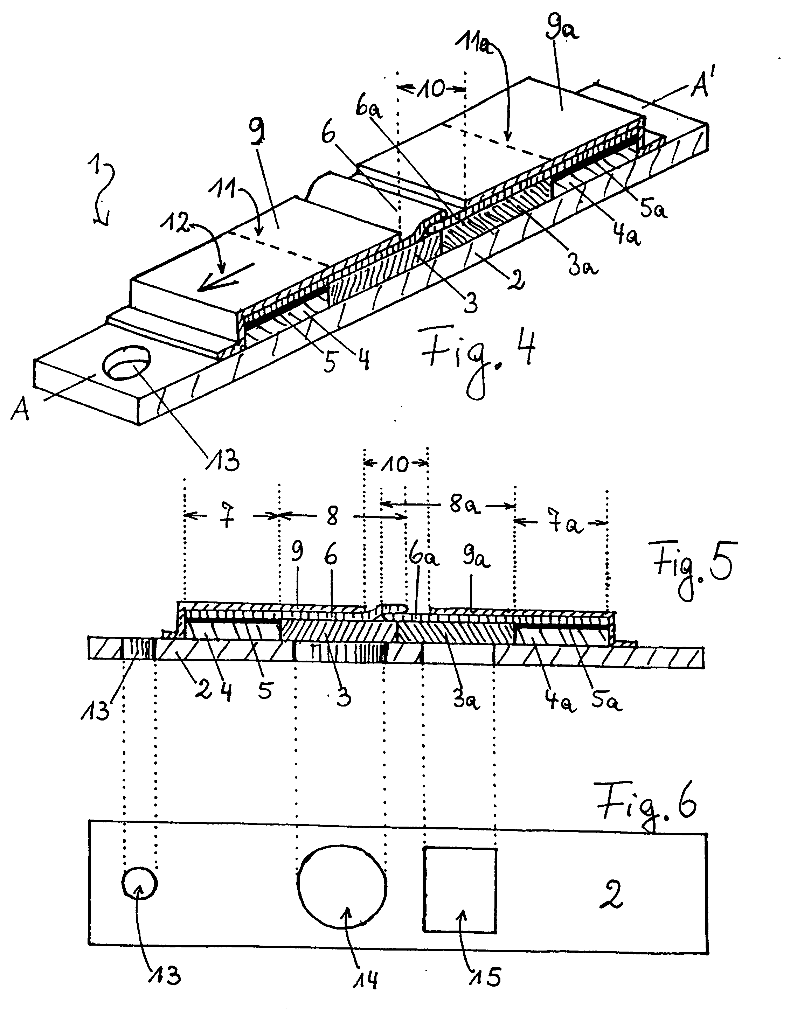

Die Figur 4 zeigt eine perspektivische Aufsicht, die Figur 5 einen Schnitt längs der Schnittlinie A-A' in Figur 4, die Figur 6 eine Aufsicht auf die Unterseite einer Ausführungsform eines erfindungsgemäßen Teststreifens mit zwei unmittelbar aneinander grenzenden Nachweisflächen und einer erfindungsgemäßen Spreitauflage, die in der oben beschriebenen besonders bevorzugten Art der einseitigen Befestigung über den Nachweisschichten fixiert ist. Auch diese Darstellung erfolgt ohne Maßstab um den Aufbau klar ersichtlich machen zu können. Eine konkrete Dimensionierung dieser Ausführungsform kann dem Ausführungsbeispiel 2 entnommen werden.

Der in Fig. 4 perspektivisch, in Fig. 5 im Schnitt und in Fig. 6 von unten gezeigte erfindungsgemäße diagnostische Teststreifen (1) weist auf einer Tragschicht (2), unmittelbar aneinander grenzend, zwei Nachweisschichten (3,3a) auf, die durch die Spreitauflage-Elemente (6,6a) überdeckt sind. Neben den Nachweisschichten (3,3a) sind die Auflageelemente (6,6a) mittels Abstandhaltern (4,4a) und Adhäsionsschichten (5,5a) auf der Tragschicht (2) befestigt. Diese Abstandhalter können in der Praxis auch Schmelzkleberflächen oder zweiseitig klebende Bänder sein, die die Spreitauflage-Elemente (6,6a) auf der Tragschicht (2) fixieren. Idealerweise besitzen die Abstandhalter mit ihren Klebeflächen in etwa die gleiche Dicke wie die Nachweisschichten (3,3a). Der hier dargestellte Aufbau weist ferner Abdeckungen (9,9a) auf, die auf der Tragschicht (2) und den Spreitauflagen (6,6a) befestigt sind. Sie sind so angeordnet, daß sie die über die Nachweisschichten (3,3a) hinausragenden Bereiche und einen Teil der über den Nachweisschichten liegenden Fläche der Auflagen (6,6a) überdecken. Sie lassen jedoch den über der Grenze der Nachweisfelder liegenden Überlappungsbereich der Auflageelemente (6,6a) frei. Dieser Bereich stellt die Probenauftragsstelle (10) dar. Hierauf wird die zu untersuchende Probenflüssigkeit aufgegeben. Sofern die Abdeckungen transparent sind, können auf ihnen noch über den äußeren Begrenzungen der Nachweisfelder Markierungen (11,11a) angebracht werden, an denen der Anwender erkennen kann, ob die Auflagen die Nachweisfelder vollständig mit probenflüssigkeit gesättigt haben. Ist dies der Fall, so hat die Probenmenge ausgereicht, andernfalls besteht der Verdacht, daß die Probenmenge zu gering war und möglicherweise eine Fehlmessung erfolgt. Die linke Abdeckung (9) enthält aufgedruckt Pfeile (12), die dem Anwender zeigen, mit welchem Ende der Testträger (1) in ein Meßgerät gelegt oder geschoben werden soll. Das Positionierloch (13) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (13) hineinragt und so den Testträger (1) an einer vorbestimmten Stelle festhält.

Die Figur 6 zeigt die Unterseite des erfindungsgemäßen Teststreifens mit der im Träger (2) angebrachten Positionierungsbohrung (13) und den unterschiedlich geformten Beobachtungsöffnungen (14 und 15), durch die die Nachweisschichten inspiziert und vermessen werden können.

In einem erfindungsgemäßen diagnostischen Testträger kommen für die Tragschicht insbesondere solche Materialien in Frage, die die zu untersuchenden Flüssigkeiten nicht aufnehmen. Dies sind sogenannte nicht-saugfähige Materialien, wobei Kunststoffolien beispielsweise aus Polystyrol, Polyvinylchlorid, Polyester, Polycarbonat oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetat verwendet werden können. Als weitere Trägermaterialien eignen sich Metallfolien oder Glas.

Für eine Nachweisschicht ist es im Gegensatz hierzu erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke, Membranen oder sonstige poröse Kunststoffmaterialien oder quellfähige Materialien, wie Gelatine- oder Dispersionsfilme, die als Schichtmaterialien verwendet werden können. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf oder in einer Schicht. Es sind jedoch auch Fälle vorstellbar, für die es vorteilhafter ist, die Reagenzien auf mehrere saug- oder quellfähige Materialschichten zu verteilen, die dann übereinander, sich vollflächig berührend angeordnet sind. Der im folgenden verwendete Begriff "Nachweisschicht" soll sowohl solche Fälle umfassen, bei denen sich die Reagenzien entweder nur in oder auf einer Schicht oder in zwei oder noch mehr, wie vorstehend beschrieben, angeordneten Schichten befinden.

Bevorzugte Materialien für die Nachweisschicht sind Papiere oder poröse Kunststoffmaterialien, wie Membranen. Hiervon besonders bevorzugt sind asymmetrisch poröse Membranen, die vorteilhafterweise so angeordnet sind, daß die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen ganz besonders bevorzugt. Hervorragend geeignet sind insbesondere Polyamid 66-Membranen und hydrophilisierte asymmetrische Polysulfonmembranen. Die Reagenzien zur Bestimmung des nachzuweisenden Analyts sind in der Regel durch Imprägnierung in die vorstehend genannten Materialien eingebracht oder durch Beschichtung einseitig aufgebracht worden. Bei Beschichtung asymmetrischer Membranen wird vorteilhafterweise die feinporige Seite beschichtet.

Für die Nachweisschicht kommen jedoch auch sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können sind dem Fachmann bekannt. Dies muß hier nicht näher ausgeführt werden.

Ein weiteres Beispiel für eine erfindungsgemäß bevorzugte Nachweisschicht ist eine Filmschicht, wie sie in WO-A-92 15 879 beschrieben ist. Diese Schicht wird aus einer Dispersion oder Emulsion eines polymeren Filmbildners hergestellt, welche zusätzlich in homogener Verteilung ein Pigment, ein Quellmittel und das Nachweisreagenz enthält. Als polymere Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z.B. von Butadien, Styrol oder Maleinsäureester geeignet. Titandioxid ist ein für den Film besonders geeignetes Pigment. Das verwendete Quellmittel soll besonders gute Quelleigenschaften aufweisen, wobei Methylvinylethermaleinsäure-Copolymer besonders empfohlen wird. Welche Reagenzien zur Bestimmung eines bestimmten Analyts eingesetzt werden, bleibt dem Fachmann überlassen.

Ganz besonders bevorzugt wird in einem erfindungsgemäßen diagnostischen Testträger ein Testfeld als Nachweisschicht eingesetzt, das aus zwei Schichten aufgebaut ist. Dieses Testfeld umfaßt eine transparente Folie, auf die in dieser Reihenfolge eine erste und eine zweite Filmschicht übereinanderliegend aufgebracht sind. Wesentlich ist, daß die auf der transparenten Folie befindliche erste Schicht im feuchten Zustand bedeutend weniger lichtstreuend ist als die darüberliegende zweite Schicht. Die nicht beschichtete Seite der transparenten Folie wird als Nachweisseite bezeichnet und die Seite der zweiten Schicht, die der Seite gegenüberliegt, mit der die zweite Schicht auf der ersten aufliegt, wird als Probenaufgabenseite bezeichnet.

Die Filmschichten werden aus Dispersionen oder Emulsionen polymerer Filmbildner hergestellt. Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z. B. von Butadien, Styrol oder Maleinsäureester geeignet.

In dem Testfeld befinden sich die zwei genannten Filmschichten auf einer transparenten Folie. Hierfür kommen insbesondere solche Kunststofffolien in Betracht, die flüssigkeitsundurchlässig sind. Polycarbonatfolie hat sich als besonders bevorzugt erwiesen.

Die beiden Filmschichten können aus Beschichtungsmassen hergestellt werden, die den gleichen polymeren Filmbildner enthalten oder sie können aus Beschichtungsmassen erzeugt werden, die unterschiedliche polymere Filmbildner enthalten.

Sofern besondere Testaufgaben und/oder Testbedingungen vorliegen, wie z.B. bei der Bestimmung von Glukose in Vollblut, ist es zweckmäßig, die Schichten so auszubilden, daß sie außer guter Erythrocytenseparation auch optische Merkmale aufweisen, die die Beobachtung der Nachweisreaktion erleichtern und die Exaktheit der Beurteilung und der meßtechnischen Erfassung verbessern.

Hierzu enthält die erste Schicht zweckmäßigerweise ein Quellmittel und gegebenenfalls einen schwach lichtstreuenden Füllstoff, die zweite Schicht ein Quellmittel und wenigstens ein stark lichtstreuendes Pigment. Daneben kann die zweite Schicht auch nicht-poröse Füllstoffe sowie poröse Füllstoffe, wie Kieselgur, in geringen Mengen enthalten, ohne dadurch für Erythrozyten durchlässig zu werden.

Durch Zugabe eines gut quellenden Quellmittels (das heißt, einer Substanz, die unter Aufnahme von Wasser ihr Volumen vergrößert) erhält man nicht nur Schichten, die relativ schnell von Probenflüssigkeit penetriert werden, sondern die trotz dieser Öffnungswirkung des Quellmittels gute Erythrozyten- und außerdem auch Blutfarbstoffabtrenneigenschaften besitzen. Die Quelleigenschaften sollten so gut sein, daß für einen Test, bei dem die Geschwindigkeit der Farbbildung - wie beispielsweise einer Glucosenachweisreaktion - überwiegend von der Penetration der Probenflüssigkeit durch die Schicht abhängt, die optisch nachweisbare Reaktion nach maximal einer Minute meßbar ist. Als besonders geeignete Quellmittel haben sich Xanthangum und Methylvinylethermaleinsäure-Copolymer erwiesen.

Kieselgur wird auch als Diatomeenerde bezeichnet. Es handelt sich um aus den Kieselsäuregerüsten der Diatomeenarten entstandene Ablagerungen, die an verschiedenen Orten abgebaut werden. Die bevorzugt eingesetzte Kieselgur hat einen mittleren Teilchendurchmesser von 5-15 μm, wobei diese Werte mit einem Laser-Granulometer Typ 715 bestimmt wurden, welches von der Firma Pabisch, München, Bundesrepublik Deutschland, vertrieben wird.

Der stark lichtstreuende Pigmentanteil der zweiten Schicht liegt bei mindestens 25 Gewichts-%, bezogen auf die getrocknete und einsatzbereite Doppelschicht des Testfeldes. Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente.

Die erste Filmschicht soll entweder keine oder solche Füllstoffe enthalten, deren Brechungsindex nahe beim Brechungsindex von Wasser liegt. Als besonders geeignet hierfür haben sich gefällte Kieselsäuren, Siliziumdioxid, Silikate und Aluminiumsilikate erwiesen. Ein Natriumaluminiumsilikat mit dem Handelsnamen Transpafill® ist besonders bevorzugt.

Die zweite Schicht soll möglichst stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 μm haben sich als besonders vorteilhaft erwiesen. Leicht verarbeitbare Titandioxid-Typen in der Anatas-Modifikation sind ganz besonders bevorzugt.

Reagenzsysteme zum Nachweis bestimmter Analyte durch Farbbildung sind dem Fachmann bekannt. Es ist möglich, daß sich sämtliche Komponenten des Reagenzsystems in einer Filmschicht befinden. Es ist aber auch möglich, daß die Komponenten des Reagenzsystems auf beide Filmschichten verteilt sind. Vorteilhafterweise befindet sich das farbbildende Reagenzsystem wenigstens zum Teil in der ersten Filmschicht.

Unter Farbbildung wird im Rahmen der vorliegenden Erfindung nicht nur der Übergang von weiß nach farbig verstanden, sondern auch jede Farbveränderung, wobei natürlich solche Farbveränderungen besonders bevorzugt sind, die mit einer möglichst großen Verschiebung der maximalen Absorptionswellenlinie (λ max) einhergehen.

Für die Optimierung des Testfeldes in dem erfindungsgemäßen diagnostischen Testträger hat es sich als besonders vorteilhaft erwiesen, wenn beide Filmschichten ein nicht hämolysierendes Netzmittel enthalten. Neutrale, das heißt, nicht geladene Netzmittel sind hierfür besonders geeignet. Ganz besonders bevorzugt wird N-Octanoyl-N-methyl-glucamid.

Zusätzlich können in den Filmschichten weitere Netzmittel, die die Homogenität der Beschichtungen fördern, wie beispielsweise Natrium-N-methyl-N-oleoyl-taurat enthalten sein.

Zur Herstellung eines Testfeldes eines erfindungsgemäßen diagnostischen Testträgers werden die jeweiligen Filmschichten jeweils nacheinander aus einer homogenen Dispersion der genannten Bestandteile hergestellt. Hierzu verwendet man als Unterlage für das Ausformen der Beschichtungsmasse für die erste Filmschicht die transparente Folie. Nach Aufbringen der Beschichtungsmasse für die erste Filmschicht in einer bestimmten Schichtdicke wird die Schicht getrocknet. Danach wird auf diese Schicht die Beschichtungsmasse für die zweite Schicht ebenfalls in einer dünnen Schichtdicke aufgebracht und getrocknet. Nach dem Trocknen sollte die Dicke der ersten und zweiten Filmschicht zusammen maximal 0,2 mm, bevorzugt maximal 0,12 mm, besonders bevorzugt maximal 0,08 mm betragen.

Die Befestigung kann nach dem Fachmann aus der Testträgertechnologie bekannten Methoden erfolgen. Beispielsweise kann die Befestigung mittels Schmelzkleber oder härtendem Kaltkleber erfolgen. Dabei ist eine punktuelle oder gerasterte Verklebung vorteilhaft, da der kapillaraktive Flüssigkeitstransport in diesem Fall besonders gut möglich ist. Als vorteilhaft haben sich auch doppelseitig klebende Streifen erwiesen. In allen Fällen ist es jedoch wichtig, daß die Befestigung der Auflage auf der Tragschicht so erfolgt, daß von der Nachweisschicht ein kapillaraktiver Flüssigkeitstransport in den Teil der Auflage möglich ist, der auf der Tragschicht befestigt ist. Dieser kapillaraktive Flüssigkeitstransport muß insbesondere dann möglich sein, wenn die Nachweisschicht mit Flüssigkeit gesättigt ist. Für die Verarbeitung ganz besonders geeignet haben sich Klebebänder mit Natur- oder Synthesekautschuk erwiesen. Ganz besonders vorteilhaft ist es, wenn das Mittel, das zur Befestigung der Auflage auf der Tragschicht dient, etwa die gleiche Dicke wie die Nachweisschicht(en) hat. Es dient dann quasi als Abstandshalter, um die erfindungsgemäße Auflage auch außerhalb des Bereiches der Nachweisschicht(en) insgesamt auf einer durchgehenden Fläche eben zu halten.

Zur Bestimmung des in der Probenflüssigkeit nachzuweisenden Analyts ist in dem erfindungsgemäßen diagnostischen Testträger die Nachweisschicht, zumindest aber sind die Reaktionsbezirke, das heißt, Reagenz tragende Bereiche der Nachweisschicht(en), die auf Signalbildung hin beobachtet und vermessen werden können, durch die Tragschicht sichtbar. Dies kann, wie oben bereits ausgeführt, dadurch erreicht werden, daß die Tragschicht transparent ist. Es ist aber auch möglich, daß die Tragschicht eine Lochung aufweist, die von der Nachweisschicht oder den Nachweisschichten überdeckt ist. Durch die Lochung ist dann die Nachweisschicht oder sind die Nachweisschichten zumindest aber die Reaktionsbezirke der Nachweisschichten sichtbar. In einer bevorzugten Ausführungsform des erfindungsgemäßen diagnostischen Testträgers befindet sich in der Tragschicht unterhalb einer Nachweisschicht ein Loch, durch das die Nachweisschicht oder ein Reaktionsbezirk beobachtbar ist. Das Loch besitzt einen etwas kleineren Durchmesser als die kleinste Längenausdehnung der Nachweisschicht, so daß die Nachweisschicht außerhalb des Loches auf der Tragschicht aufliegt und dort befestigt sein kann. Vorteilhafterweise ist die Nachweisschicht durch beidseitig daneben angeordnete doppelseitige Klebebänder und die über der Nachweisschicht liegende erfindungsgemäße Auflage und deren Befestigung auf der Tragschicht ausreichend fixiert. Bevorzugt ist jedoch die Nachweisschicht selbst auch mittels dünnem Klebeband auf der Tragschicht befestigt.

Durch ein Loch können aber auch mehrere Reaktionsbezirke einer Nachweisschicht sichtbar sein.

Die Lochung eines erfindungsgemäßen diagnostischen Testträgers kann auch aus zwei oder mehr Löchern bestehen, die zur Bestimmung von Analyt (einem oder mehreren Analyten) genutzt werden können. Über den Löchern können verschiedene Nachweisschichten angeordnet sein oder auch nur eine Nachweisschicht mit mehreren Reaktionsbezirken, so daß durch je 1 Loch eine Nachweisschicht oder je ein Reaktionsbezirk beobachtet werden kann. Es ist auch möglich, daß durch ein Loch mehrere Reaktionsbezirke beobachtet werden können.

Über der erfindungsgemäßen Spreitauflage des erfindungsgemäßen diagnostischen Testträgers kann zweckmäßigerweise eine inerte Abdeckung aus probenundurchlässigem, in der Regel wasserundurchlässigem und nicht saugfähigem Material, so angeordnet werden, daß der Bereich der Auflage außerhalb der Nachweisschicht abgedeckt ist. Idealerweise ragt die Abdeckung auch noch in den Bereich der Nachweisschicht hinein, läßt jedoch auf jeden Fall einen mittleren Teil der erfindungsgemäßen Auflage, die die Nachweisschicht bedeckt, frei. Dieser freie Teil der Auflage wird als Probenauftragsstelle bezeichnet.

Als Abdeckung haben sich Kunststoffolien als besonders vorteilhaft erwiesen. Wenn Abdeckung und die erfindungsgemäße Auflage unterschiedliche Farben haben, beispielsweise weiß und gelb oder weiß und rot, kann damit die Stelle sehr gut kenntlich gemacht werden, auf die zu untersuchende Probenflüssigkeit aufgegeben werden soll.

Auf der Abdeckung kann auch beispielsweise mit einem oder mehreren aufgedruckten Pfeilen verdeutlicht werden, in welcher Richtung, das heißt, mit welchem Ende ein erfindungsgemäßer diagnostischer Testträger in ein Meßgerät gelegt oder geschoben werden soll.

Eine Probenauftragsstelle kann besonders einfach durch eine Abdeckung mittels zweier bandförmiger Kunststoffolien erreicht werden, die einen bandartigen Bereich der die Nachweisschicht überdeckenden erfindungsgemäßen Auflage frei lassen. Wenn 2 oder mehr Probenauftragsstellen vorgesehen werden, sind 3 oder mehr bandförmige Kunststoffolien zu verwenden. Die zur Abdeckung verwendeten Folien sind auf der erfindungsgemäßen Auflage und gegebenenfalls auf der Tragschicht befestigt. Für eine solche Befestigung eignen sich Schmelzkleber, die vorzugsweise punktuell oder gerastert auf der Tragschicht oder der Unterseite der Abdeckung aufgebracht sind oder Klebebänder, wenn die Folien nicht selbst klebefähig sind. Die Probenauftragsstelle befindet sich vorzugsweise über der Lochung in der Tragschicht, durch die eine Signalbildung in der Nachweisschicht beobachtet werden kann.

Zur Durchführung eines Verfahrens zur Bestimmung von Analyt in flüssiger Probe mit Hilfe eines erfindungsgemäßen diagnostischen Testträgers wird Probenflüssigkeit auf die der Nachweisschicht abgewandten Seite der Auflage aufgegeben, idealerweise so viel, daß die durch die erfindungsgemäße Auflage gelangende Flüssigkeit die Nachweisschicht vollständig sättigt. Als Probenflüssigkeit kommen insbesondere Körperflüssigkeiten wie Blut, Plasma, Serum, Urin, Speichel etc. in Frage. Blut oder von Blut abgeleitete Flüssigkeiten wie Plasma oder Serum sowie Urin sind besonders bevorzugte Probenflüssigkeiten. In der Nachweisschicht kann dann bei Anwesenheit des zu bestimmenden Analyts ein Signal nachgewiesen werden. Vorteilhafterweise handelt es sich bei einem solchen Signal um eine Farbänderung, worunter sowohl Farbbildung, Farbverlust als auch Farbumschlag verstanden wird. Die Intensität der Farbänderung ist ein Maß für die Menge an Analyt in der untersuchten flüssigen Probe. Sie kann visuell oder mit Hilfe eines Gerätes, meistens reflektionsphotometrisch quantitativ ausgewertet werden, wobei in Vorversuchen geschaffene Eichkurven benutzt werden können. Alternativ kann auch eine direkte Anzeige des Analytgehalts über die Geräte-Software bewerkstelligt werden.

Ein großer Vorteil des erfindungsgemäßen diagnostischen Testträgers besteht darin, daß kein vorbestimmtes Volumen einer Probenflüssigkeit auf den Testträger aufgegeben werden muß.

Es hat sich nämlich gezeigt, daß bei der Verwendung eines Teststreifens, der unter Einsatz der oben genannten bevorzugten Materialien konstruiert wurde, ein Probenüberschuß von dem Streifen nicht aufgenommen wird sondern über der Auftragsstelle stehen bleibt. Ein weiterer erheblicher Vorteil der erfindungsgemäßen Konstruktionen ist es, daß der Teststreifen "selbstdosierend" ist. Bringt man seine Auftragsstelle mit einem auf der Fingerbeere stehenden oder an ihr hängenden Blutstropfen in Berührung, so entnimmt sich der Streifen nur die Menge, die zur Durchtränkung der Nachweisschicht(en) erforderlich ist, der Rest bleibt am Finger.

Auf diese Art und Weise ist die bei Anwesenheit eines Analyts sich einstellende Signalintensität unabhängig von der Menge und der Dauer des Kontaktes der Probenflüssigkeit mit der Nachweisschicht. Die Farbe, die sich nach Beendung der Nachweisreaktion, üblicherweise innerhalb weniger Sekunden bis weniger Minuten eingestellt hat, bleibt so für die Messung unverändert. Sie wird lediglich durch die Stabilität des farbgebenden Systems bestimmt. Falsch positive Resultate werden so ebenfalls vermieden und eine quantitative Analytbestimmung ermöglicht.

Durch die Abdeckung von Teilen der erfindungsgemäßen Auflage und damit der Markierung der Probenauftragsstelle wird dafür Sorge getragen, daß Flüssigkeit nur an der dafür optimalen Stelle auf die Nachweisschicht gelangen kann. In Kombination mit einer Nachweisschicht, die nur wenig Flüssigkeit aufnimmt und dennoch eine intensive Signalbildung gewährleistet, wird sichergestellt, daß bereits bei sehr kleinen Probenvolumina zuverlässige Analytbestimmungen möglich sind. Dadurch, daß der erfindungsgemäße Testträger aus nur wenigen Komponenten besteht, die einfach und schnell zusammenfügbar sind, ist er sehr preiswert herzustellen.

Die folgenden Ausführungsbeispiele veranschaulichen die Herstellung erfindungsgemäßer Spreitauflagen und Testreifen.

**Beispiel 1**

A.) Herstellung einer erfindungsgemäßen Spreitauflage.

A.1) Zu 55 kg destilliertem Wasser werden 55,0 g N-Oleoyl-sarcosin ("®Crodasinic O" der Fa. Croda, Nettetal) gegeben und unter Rühren durch Zugabe von 11,0 g 32 gew.-%iger Natronlauge auf pH 6,0 gestellt. Man erhält eine erfindungsgemäß einzusetzende Netzmittellösung mit einem Gehalt von ca. 0,106 Gew.-% Wirksubstanz.

A.2) Durch diese Lösung wird ein 1 m breites, 700 m langes ®Viledon-Vlies, Typ FO 2451/121, der Fa. Freudenberg, Weinheim, (Dicke 50 µm, Flächengewicht 18 g/m$^2$) mit einer Geschwindigkeit von 5 m/min gezogen. Das getränkte Vlies wird anschließend in einem Horizontaltrockner von 30 m Länge bei 80°C und einem Luftdurchsatz von 50 m$^3$/min getrocknet. Die Flottenaufnahme des Vlieses betrug 45 ml/m$^2$, sodaß der Gehalt an Wirksubstanz auf dem imprägnierten Vlies ca. 0,26 Gew.-% beträgt.

B.) Herstellung erfindungsgemäßer Teststreifen.

B.1) Auf eine bandförmige, 50 mm breite Titandioxid-haltige Polyester-Tragschicht wird parallel im Abstand von 18,6 mm (gemessen zur linken Kante des Klebebandes) zu seiner linken Kante ein 5 mm breites doppelseitiges Klebeband (Polyesterträger und Synthesekautschuk-Kleber) aufgebracht. Aus diesem Verbund werden mit einem Abstand von 6 mm jeweils zwei Löcher, ein Positionierungsloch und ein Inspektions- und Meßloch, ausgestanzt, deren Mittelpunkte auf einer senkrecht zur Längachse der Trägerstreifens liegenden Geraden liegen. Das erste Loch, das Positionierungsloch, ist kreisrund, hat einen Durchmesser von 2,6 mm und sein Mittelpunkt hat von der linken Kante des Trägerstreifens einen Abstand von 4 mm. Das zweite Loch ist ebenfalls rund mit einem Durchmesser von 4 mm. Der Mittelpunksabstand des zweiten Loches von der linken Kante des Trägerstreifens beträgt 21 mm.

Danach wird das Schutzpapier des doppelseitigen Klebebands abgezogen.

Zur Herstellung einer Nachweisschicht, die aus 2 Filmschichten aufgebaut ist, wird so vorgegangen:

B.2) In einem Becherglas werden die folgenden Komponenten als Reinsubstanzen oder in Form von Stammlösungen in folgender Zusammensetzung zusammengegeben und durch Rühren gemischt:

Wasser: 820,0 g

Citronensäure-1-hydrat: 2,5 g

Calciumchlorid-2-hydrat: 0,5 g

Natriumhydroxid: 1,4 g

Xanthan gum: 3,4 g

Tetraethylammoniumchlorid: 2,0 g

Natrium-N-methyl-N-oleoyl-taurat: 0,29 g

N-Octanoyl-N-methyl-glucamid: 2,1 g

Polyvinylpyrrolidon (MG 25000): 3,5 g

Transpafill( (Natrium-Aluminiumsilikat): 62,1 g

Polyvinylpropionat-Dispersion (50 Gew.-% in Wasser): 60,8 g

Bis-(2-hydroxyethyl)-(4-hydroximinocyclohexa-2,5-dienylidin)-ammoniumchlorid: 1,2 g

2,18-Phosphormolybdänsäure-hexanatriumsalz: 16,1 g

Pyrrolochinolin-chinon: 32 mg

Glucosedehydrogenase rec. aus

Acinetobacter calcoaceticus, 1,7 MU EC 1.1.99.17: (2,4 g)

1-Hexanol: 1,6 g

1-Methoxy-2-propanol: 20,4 g

Die Gesamtmasse wird mit NaOH auf einen pH von ca. 6 eingestellt und dann mit einem Flächengewicht von 89 g/m$^2$ auf eine 125 µm dicke Polycarbonatfolie aufgetragen und getrocknet.

B.3) In einem Becherglas werden die folgenden Komponenten als Reinsubstanzen oder in Form von Stammlösungen in folgender Zusammensetzung zusammengegeben und durch Rühren gemischt:

Wasser: 579,7 g

Natriumhydroxid: 3,4 g

Gantrez( (Methylvinylether-maleinsäure-Copolymer): 13,8 g

Natrium-N-methyl-N-oleoyl-taurat: 0.25 g

N-Octanoyl-N-methyl-glucamid: 3,6 g

Tetraethylammoniumchlorid: 9,7 g

Polyvinylpyrrolidon (MG 25000): 20,2 g

Titandioxid: 177,1 g

Kieselgur: 55,3 g

Polyvinylpropionat-Dispersion (50 Gew.-% in Wasser): 70,6 g

2,18-Phosphormolybdänsäure-hexanatriumsalz: 44,3 g

Kaliumhexacyanoferrat (III): 0,3 g

1-Hexanol: 1,6 g

1-Methoxy-2-propanol: 20,4 g

Die Gesamtmasse wird mit NaOH auf einen pH von ca. 6 eingestellt und dann mit einem Flächengewicht von 104 g/m$^2$ auf die wie vorstehend unter A. beschriebene beschichtete Polycarbonatfolie aufgetragen und getrocknet.

B.4) Ein 5 mm breiter Streifen der so hergestellten Nachweisschicht wird mit der Folienseite auf das gestanzte doppelseitige Klebeband auf der Tragschicht passgenau aufgeklebt.

Direkt an die Nachweisschicht angrenzend werden auf beiden Seiten doppelseitige Klebebänder (PVC-Träger und Naturkautschuk-Kleber) als Abstandshalter auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Auf diesen Verbund wird ein 20 mm breiter Streifen des in Abschnitt A hergestellten Spreitvlieses aufgelegt und durch Anpressen verklebt.

Es werden zwei einseitige Klebebänder (PVC-Träger und Naturkautschuk-Kleber) als Abdeckungen so auf das Spreitvlies aufgeklebt, daß die Abstandshalter ganz abgedeckt werden und wenigstens noch eine geringfügige Überlappung mit dem Reaktivbezirk stattfindet. Damit ist die Bandware fertiggestellt.

Die Bandware wird so in 6 mm breite Testträger geschnitten, daß das Meßloch im Testträger mittig liegt.

**Beispiel 2**

Herstellung eines erfindungsgemäßen diagnostischen Testträgers mit zwei Nachweisfeldern für die Bestimmung von Glucose in niedriger und in hoher Konzentration.

Die Herstellung eines Testträgers gemäß den Figuren 4, 5 und 6 erfolgt mit folgenden Arbeitsschritten:

Auf eine bandförmige, 50 mm breite Titandioxid-haltige Polyester-Tragschicht wird parallel im Abstand von 18,6 mm (gemessen zur linken Kante des Klebebandes) zu seiner linken Kante ein 10 mm breites doppelseitiges Klebeband (Polyesterträger und Synthesekautschuk-Kleber) aufgebracht. Aus diesem Verbund werden mit einem Abstand von 6 mm jeweils drei Löcher, ein Positionierungsloch und zwei Inspektions- und Meßlöcher, ausgestanzt, deren Mittelpunkte auf einer senkrecht zur Längachse der Trägerstreifens liegenden Geraden liegen. Das erste Loch, das Positionierungsloch, ist kreisrund, hat einen Durchmesser von 2,6 mm und sein Mittelpunkt hat von der linken Kante des Trägerstreifens einen Abstand von 4 mm. Das zweite Loch ist ebenfalls rund mit einem Durchmesser von 4 mm, das dritte Loch ist rechteckig mit einer Kantenlänge von 3 mm in Längsrichtung des Streifens und 4 mm in Querrichtung. Das zweite und dritte Loch liegen beide in einem Mittelpunktsabstand von 5,1 mm auf dem Klebeband der Mittelpunksabstand des zweiten Loches von der linken Kante des Trägerstreifens beträgt 21 mm.

Danach wird das Schutzpapier des doppelseitigen Klebebands abgezogen.

Zur Herstellung der ersten Nachweisschicht, die aus 2 Filmschichten aufgebaut ist, wird so vorgegangen:

A. In einem Becherglas werden die folgenden Komponenten als Reinsubstanzen oder in Form von Stammlösungen in folgender Zusammensetzung zusammengegeben und durch Rühren gemischt:

Wasser: 820,0 g

Citronensäure-1-hydrat: 2,5 g

Calciumchlorid-2-hydrat: 0,5 g

Natriumhydroxid: 1,4 g

Xanthan gum: 3,4 g

Tetraethylammoniumchlorid: 2,0 g

N-Octanoyl-N-methyl-glucamid: 2,1 g

Polyvinylpyrrolidon (MG 25000): 3,5 g

Transpafill( (Natrium-Aluminiumsilikat): 62,1 g

Polyvinylpropionat-Dispersion (50 Gew.-% in Wasser): 60,8 g

Bis-(2-hydroxyethyl)-(4-hydroximinocyclohexa-2,5-dienylidin)-ammoniumchlorid: 1,2 g

2,18-Phosphormolybdänsäure-hexanatriumsalz: 16,1 g

Pyrrolochinolin-chinon: 32 mg

Glucosedehydrogenase rec. aus Acinetobacter calcoaceticus, 1,7 MU EC 1.1.99.17: (2,4 g)

1-Hexanol: 1,6 g

1-Methoxy-2-propanol: 20,4 g

Die Gesamtmasse wird mit NaOH auf einen pH von ca. 6 eingestellt und dann mit einem Flächengewicht von 89 g/m$^2$ auf eine 125 µ dicke Polycarbonatfolie aufgetragen und getrocknet.

B. In einem Becherglas werden die folgenden Komponenten als Reinsubstanzen oder in Form von Stammlösungen in folgender Zusammensetzung zusammengegeben und durch Rühren gemischt:

Wasser: 579,7 g

Natriumhydroxid: 3,4 g

Gantrez( (Methylvinylether-maleinsäure-Copolymer): 13,8 g

N-Octanoyl-N-methyl-glucamid: 3,6 g

Tetraethylammoniumchlorid: 9,7 g

Polyvinylpyrrolidon (MG 25000): 20,2 g

Titandioxid: 177,1 g

Kieselgur: 55,3 g

Polyvinylpropionat-Dispersion (50 Gew.-% in Wasser): 70,6 g

2,18-Phosphormolybdänsäure-hexanatriumsalz: 44,3 g

Kaliumhexacyanoferrat (III): 0,3 g

1-Hexanol: 1,6 g

1-Methoxy-2-propanol: 20,4 g

Die Gesamtmasse wird mit NaOH auf einen pH von ca. 6 eingestellt und dann mit einem Flächengewicht von 104 g/m$^2$ auf die wie vorstehend unter A. beschriebene beschichtete Polycarbonatfolie aufgetragen und getrocknet. Die Schichtdicke baträgt nach dem Trocknen 60 μm.

Zur Herstellung der zweiten Nachweisschicht, die ebenfalls aus 2 Filmschichten aufgebaut ist, verfährt man wie folgt:

A. In einem Becherglas werden die folgenden Komponenten als Reinsubstanzen oder in Form von Stammlösungen in den angegebenen Mengen unter Rühren gemischt:

Wasser: 820,0 g

Citronensäure-1-hydrat: 2,5 g

Calciumchlorid-2-hydrat: 0,5 g

Natriumhydroxid: 1,4 g

Xanthan gum: 3,4 g

Tetraethylammoniumchlorid: 4,22 g

N-Octanoyl-N-methyl-glucamid: 2,1 g

Natrium-N-methyl-N-oleoyl-taurat 0,29 g

Polyvinylpyrrolidon (MG 25000): 3,5 g

Transpafill( (Natrium-Aluminiumsilikat): 62,1 g

Polyvinylpropionat-Dispersion (50 Gew.-% in Wasser): 60,8 g

N-(4-Nitrosophenyl)-N'-carboxymethyl-piperazin: 1,0 g

2,18-Phosphormolybdänsäure-hexanatriumsalz: 20,9 g

Pyrrolochinolin-chinon: 32 mg

Glucosedehydrogenase rec. aus

Acinetobacter calcoaceticus, 1,7 MU (EC 1.1.99.17): (2,4 g)

1-Hexanol: 1,6 g

1-Methoxy-2-propanol: 20,4 g

Die Gesamtmasse wird mit Natronlauge auf einen pH-Wert von ca. 6,0 eingestellt und dann mit einem Flächengewicht von 89 g/m$^2$ auf eine 125 μm dicke Polycarbonatfolie aufgetragen und getrocknet.

B. In einem Becherglas werden die folgenden Komponenten als Reinsubstanzen oder in Form von Stammlösungen in den angegebenen Mengen unter Rühren gemischt:

Wasser: 579,7 g

Natriumhydroxid: 3,4 g

Gantrez( (Methylvinylether-maleinsäure-Copolymer): 13,8 g

Tetraethylammoniumchlorid: 6,71 g

N-Octanoyl-N-methyl-glucamid: 2,74 g

Natrium-N-methyl-N-oleoyl-taurat 0,25 g

Polyvinylpyrrolidon (MG 25000): 15,6 g

Titandioxid: 136,7 g

Polyvinylpropionat-Dispersion (50 Gew.-% in Wasser): 54,6 g

N-(4-Nitrosophenyl)-N'-carboxymethyl-piperazin: 1,51 g

2,18-Phosphormolybdänsäure-hexanatriumsalz: 33,13 g

Kaliumhexacyanoferrat (III): 0,28 g

1-Hexanol: 1,6 g

1-Methoxy-2-propanol: 20,4 g

Die Gesamtmasse wird mit Natronlauge auf einen pH-Wert von ca. 6,0 eingestellt und dann mit einem Flächengewicht von 102 g/m² auf die wie vorstehend unter A beschriebene beschichtete Polycarbonatfolie aufgetragen und getrocknet. Die Schichtdicke beträgt nach dem Trocknen 55 μm.

Je ein 5 mm breiter Streifen der so hergestellten Nachweisschichten wird mit der Folienseite auf das gestanzte doppelseitige Klebeband auf der Tragschicht passgenau so aufgeklebt, das die Streifen unmittelbar aneinander grenzend nebeneinander herlaufen.

Direkt an die Nachweisschichten angrenzend werden auf beiden Seiten doppelseitige Klebebänder in der Dicke der Nachweisstreifen (PVC-Träger und Naturkautschuk-Kleber) als Abstandshalter auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Dann wird ein 10 mm breiter Streifen des gemäß Abschnitt A des Beispiels 1 hergestellten Spreitvlieses so auf den 9 mm breiten Abstandshalter aufgelegt, daß die Schnittkante des Gewebestreifens die Grenzlinie zwischen den Nachweisstreifen um 0,5 bis 0,6 mm überragt und durch Andrücken fixiert. Anschließend wird auf den 6 mm breiten Abstandshalter ein 10 mm breiter Streifen des gleichen erfindungsgemäßen Spreitvlieses so aufgelegt, daß er die Schnittkante des ersten Gewebestreifens um 1 bis 1,2 mm überlappt und durch Andrücken fixiert.

Danach werden auf beiden Seiten des Aufbaus zwei einseitige Klebebänder (PVC-Träger und Naturkautschuk-Kleber) als Abdeckungen so aufgeklebt, daß symmetrisch zur Grenzlinie der Nachweisstreifen ein Spalt von 2 bis 2,5 mm unbedeckt bleibt. Damit ist die Bandware fertiggestellt.

Die Bandware wird so in 6 mm breite Testträger geschnitten, daß die Meß- und Inspektionslöcher und das Positionierungsloch im Testträger mittig liegen.

**Beispiel 3**

Gemäß Beispiel 2 werden Teststreifen hergestellt, die in beiden Nachweisfeldern die gleichen Nachweisschichten aufweisen. Als Spreitschicht wird ein mit 0,25 Gew.-% Natrium-N-oleoyl-sarcosinat imprägniertes Polyestergewebe Typ PE 38 HC eingesetzt. Die Streifen werden in ein für die gleichzeitige Ausmessung beider Testfelder eingerichtetes GLUCOTREND-Gerät eingeschoben, wobei sie zur Fixierung in der Meßposition einer leichten Biegung

**EP 0 995 992 B1**

unterworfen werden.

Die Streifen werden mit steigenden Volumina EDTA-Venenblut mit 102 mg/dl Glucose getüpfelt. Je Volumen wurden 5 Serien zu 10 Teststreifen vermessen (n=5, N=50). Hieraus wurden für jedes Volumen 5 VK-Werte berechnet. (Der VK-Wert ist definiert als die relative Standardabweichung

$$VK = \text{Standardabweichung} / \text{Mittelwert}$$

und wird in % angegeben.)

Die folgende Tabelle zeigt die Mediane der Messergebnisse und die Mediane der VK-Werte der jeweils 5 Serien je Volumen:

| Volumen | Feld 1 | | Feld 2 | |
| --- | --- | --- | --- | --- |
| | Median des Meßwertes [mg/dl] | Median des VK-Wertes [%] | Median des Meßwertes [mg/dl] | Median des VK-Wertes [%] |
| 3 µl | Fehler* | - | 63,4** | 9,3 |
| 4 µl | 100 | 2,5 | 102 | 2,7 |
| 5 µl | 102 | 2,3 | 103 | 2,6 |
| 10 µl | 101 | 2,2 | 102 | 2,7 |
| 15 µl | 103 | 2,4 | 102 | 2,7 |

Anmerkungen:

*: Eine ungleichmäßige Reaktionsfärbung wird auf dem Feld 1 durch die 2-LED-Optik des Gerätes (beschrieben in EP-A-819 943) erkannt. Das Gerät gibt daher nur eine Fehlermeldung, aber keinen Meßwert aus.

**:Feld 2 wird nur mit einer LED beleuchtet, sodaß eine ungleichmäßige Färbung des Testfeldes nicht erkannt wird. Demzufolge ist der VK-Wert stark erhöht. Das Gerät kann jedoch durch Vergleich beider Testfelder die Fehlermeldung "verschieden tiefe Reaktionsfarbe beider Felder" abgeben. Diese Funktion war bei der hier durchgeführten Messung abgeschaltet.

Die Versuche zeigen, daß ab einem Probevolumen von 4 µl beide Testfelder die gleiche Färbung, d.h. die gleiche Glucosekonzentration anzeigen. Hieraus erkennt man die ausgezeichnete Spreitwirkung der erfindungsgemäßen Auflage über beide Meßfelder. Bei höheren Probevolumina ändert sich der Wert nicht, da das überschüssige Probenmaterial über dem Auftragsspalt stehen bleibt. Bei kleineren Volumina ist die Benetzung und damit auch die Färbung der beiden Reaktionsfelder unvollständig, was durch die 2-LED-Optik des Feldes 1 detektiert werden kann. Durch geeignete Softwaremaßnahmen kann verhindert werden, daß solche Messungen zu einer Anzeige von (falsch-negativen) Werten führen. Stattdessen wird eine Fehlermeldung angezeigt, die den Benutzer auf das zu niedrige Probenvolumen hinweist.

**Patentansprüche**

1. Spreitmaterial umfassend ein mit einem Netzmittel imprägniertes poröses Flächengebilde, **dadurch gekennzeichnet, daß** das Netzmittel Natrium-N-oleoyl-sarcosinat ist.

2. Spreitmaterial gemäß Anspruch 1, **dadurch gekennzeichnet, daß** das Spreitmaterial 0,01 bis 2,0 Gew.-%, vorzugsweise von 0,03 bis 0,5 Gew.-% von Natrium-N-oleoyl-sarcosinat bezogen auf das Gewicht des Materials vor der Imprägnierung, enthält.

3. Spreitmaterial gemäß mindestens einem der Ansprüche 1 bis 2, **dadurch gekennzeichnet, daß** das dem erfindungsgemäßen Spreitmaterial zugrundeliegende poröse Flächengebilde ein textiles Flächengebilde aus Monofilamenten oder entsprechenden Multifilamentgarnen ist.

4. Spreitmaterial gemäß mindestens einem der Ansprüche 1 bis 3, **dadurch gekennzeichnet, daß** das textile Flächengebilde ein Gewebe, Gewirke oder Vliesstoff mit einem Flächengewicht von 10 bis 200, insbesondere von 10 bis 50 g/m$^2$ ist.

5. Spreitmaterial gemäß mindestens einem der Ansprüche 1 bis 4, **dadurch gekennzeichnet, daß** das Textilmaterial eine Dicke von 20 bis 200, insbesondere 30 bis 100 µm Dicke und/oder ein Porenvolumen von 30 bis 85, insbesondere 40 bis 75 % hat.

6.  Verwendung von Natrium-N-oleoyl-sarcosinat zur Herstellung eines Spreitmaterials.

7.  Verwendung gemäß Anspruch 6, **dadurch gekennzeichnet, daß** das Natrium-N-oleoyl-sarcosinat in reiner Form oder in Form von Lösungen oder flüssigen Zubereitungen verwendet wird.

8.  Verwendung gemäß mindestens einem der Ansprüche 6 und 7, **dadurch gekennzeichnet, daß** die verwendete Zubereitung neben Natrium-N-oleoyl-sarcosinat weitere Hilf-und/oder Zusatzstoffe enthält.

9.  Verfahren zur Herstellung eines Spreitmaterials durch Imprägnierung eines porösen Flächengebildes mit einem Netzmittel oder einer Netzmittelzubereitung, gegebenenfalls Einstellung des imprägnierten Flächengebildes auf eine vorbestimmte Netzmittelaufnahme und gegebenenfalls Trocknen des Materials, **dadurch gekennzeichnet, daß** als Netzmittel Natrium-N-oleoyl-sarcosinat eingesetzt wird.

10.  Verfahren gemäß Anspruch 9, **dadurch gekennzeichnet, daß** die applizierte Menge des Natrium-N-oleoyl-sarcosinats so bemessen wird, daß auf dem porösen Material eine Auflage von 0,01 bis 2,0 Gew.-%, vorzugsweise von 0,03 bis 0,5 Gew.-% der applizierten Verbindung bezogen auf das Gewicht des Materials vor der Imprägnierung, verbleibt.

11.  Teststreifen aus einem gegebenenfalls transparenten oder mit Inspektionsöffnungen versehenen, flexiblen, flächenförmigen Träger, auf dem ein oder mehrere Testfelder nebeneinander angeordnet sind, die jeweils eine oder mehrere übereinanderliegende Nachweisschichten tragen, **dadurch gekennzeichnet, daß** die Testfelder durch eine Auflage aus einem Spreitmaterial gemäß mindestens einem der Ansprüche 1 bis 5 abgedeckt sind.

12.  Teststreifen gemäß Anspruch 11, **dadurch gekennzeichnet, daß** die Spreitauflage aus einem oder mehreren flächenförmigen Auflageelementen besteht, die auf dem Teststreifen so befestigt sind, daß ein Teil ihrer Fläche gegenüber der von diesem Teil abgedeckten Streifenfläche in Richtung einer beim Biegen des Objekts erzeugten Krümmung frei verschiebbar ist.

13.  Teststreifen gemäß Anspruch 12, **dadurch gekennzeichnet, daß** die Testfelder durch die verschiebbaren Flächenbereiche einer aus zwei Elementen bestehenden Auflage bedeckt sind.

14.  Teststreifen gemäß mindestens einem der Ansprüche 12 und 13, **dadurch gekennzeichnet, daß** die Spreitauflage aus zwei Auflageelementen besteht, deren verschiebbare Bereiche gegeneinander gerichtet sind und sich überlappen.

15.  Teststreifen gemäß mindestens einem der Ansprüche 11 bis 14, **dadurch gekennzeichnet, daß** die Überlappung der beiden Auflageelemente über der Trennungslinie zwischen den beiden Testfeldern und vorzugsweise symmetrisch dazu liegt.

16.  Teststreifen gemäß mindestens einem der Ansprüche 11 bis 15, **dadurch gekennzeichnet, daß** er zwei unmittelbar aneinandergrenzende oder durch einen Spalt getrennte ein- oder mehrschichtige Testfelder für den gleichen oder verschiedene diagnostisch verwertbare Analyte aufweist.

17.  Teststreifen gemäß mindestens einem der Ansprüche 11 bis 16, **dadurch gekennzeichnet, daß** die Anordnung von Nachweisschichten und Auflagen auf dem Teststreifen mit einem inerten flächenförmigen Material so abgedeckt ist, daß nur im Bereich der Überlappung der Auflageelemente in Richtung der Längsachse des Teststreifens gesehen, eine für den Probenauftrag ausreichende Strecke freibleibt.

18.  Teststreifen gemäß mindestens einem der Ansprüche 11 bis 17, **dadurch gekennzeichnet, daß** die Hydrophilie, die Durchlässigkeit und das Flüssigkeitsleitvermögen des Auflagematerials so abgestimmt sind, daß eine Analyt-Probe über den gesamten analytsensitiven Bereich des Testträgers verteilt wird, der Teststreifen selbstdosierend ist, bzw. überschüssige Probenmenge über dem Auftragspunkt stehen bleibt.

19.  Teststreifen gemäß Anspruch 11, **dadurch gekennzeichnet, daß** er ein Testfeld enthält, auf dem ein monofiles Spreitmaterial gemäß Ansprüchen 1 und 3 aufliegt, das größer als das Testfeld ist und beiderseits des Testfeldes auf dem Träger, vorzugsweise über einen Abstandshalter in Dicke des Testfeldes, befestigt ist, wobei der Teil des Spreitmaterials, der über das Testfeld hinausragt durch probenundurchlässiges Material abgedeckt ist, so daß ein Probenauftrag nur auf den Teil des Spreitmaterials möglich ist, der auf dem Testfeld aufliegt.

20. Verfahren zur Herstellung eines diagnostischen Teststreifens bei dem man auf einem gegebenenfalls transparenten oder mit Inspektionsöffnungen versehenen Träger ein- oder mehrschichtige Nachweisfelder aufbringt, diese mit einer seitlich von den Testfeldern fixierten auf den Testfeldern lose aufliegenden funktionellen Auflage überdeckt und gegebenenfalls noch eine inerte Abdeckung, die nur die Probenauftragsstelle freiläßt aufbringt, **dadurch gekennzeichnet, daß** man eine funktionelle Auflage einsetzt, die aus einem Textilmaterial mit den in einem der Ansprüche 1 bis 5 angegebenen Merkmalen besteht.

21. Verfahren gemäß Anspruch 20, **dadurch gekennzeichnet, daß** man ein flächenförmiges Auflageelement, oder mehrere flächenförmige Auflageelementen, die, sich überlappend, gemeinsam die Auflage bilden, auf dem Teststreifen neben dem (den) Testfeld(ern) so befestigt, daß ein Teil ihrer Fläche das (die) Testfeld(er) überdeckt und gegenüber dem (den) Testfeld(em) in Richtung der beim Biegen des Objekts erzeugten Krümmung frei verschiebbar ist.

22. Verwendung eines diagnostischen Teststreifens gemäß einem der Patentansprüche 11 bis 19 zur Bestimmung von Analyt in einer Flüssigkeit.

23. Verfahren zur Bestimmung von Analyt in einer flüssigen Probe, wobei Probenflüssigkeit auf die Probenauftragsstelle aufgegeben wird und die Nachweisschicht(en) auf eine Signalbildung hin beobachtet wird (werden), wobei die Signalbildung ein Maß für die Anwesenheit bzw. Menge an Analyt in der untersuchten flüssigen Probe darstellt, **dadurch gekennzeichnet, daß** ein diagnostischer Teststreifens gemäß einem der Patentansprüche 11 bis 19 eingesetzt wird.

**Claims**

1. Spreading material comprising a porous flat-shaped structure impregnated with a wetting agent, wherein the wetting agent is sodium N-oleoyl-sarcosinate.

2. Spreading material as claimed in claim 1, wherein the spreading material contains 0.01 to 2.0 % by weight, preferably 0.03 to 0.5 % by weight sodium N-oleoyl-sarcosinate relative to the weight of the material before impregnation.

3. Spreading material as claimed in at least one of the claims 1 to 2, wherein the underlying porous flat-shaped structure of the spreading material according to the invention is a textile sheet material made of monofilaments or corresponding multifilament yarns.

4. Spreading material as claimed in at least one of the claims 1 to 3, wherein the textile flat-shaped structure is a fabric, knitted fabric or fleece material with a weight per unit area of 10 to 200, in particular 10 to 50 $g/m^2$.

5. Spreading material as claimed in at least one of the claims 1 to 4, wherein the textile material has a thickness of 20 to 200, in particular 30 to 100 $\mu$m and/or a pore volume of 30 to 85, in particular 40 to 75 %.

6. Use of sodium N-oleoyl-sarcosinate to produce a spreading material.

7. Use as claimed in claim 6, wherein the sodium N-oleoyl-sarcosinate is used in a pure form or in the form of solutions or liquid formulations.

8. Use as claimed in at least one of the claims 6 and 7, wherein the formulation used contains auxiliary substances and/or additives in addition to sodium N-oleoyl-sarcosinate.

9. Process for the production of a spreading material by impregnating a porous flat-shaped structure with a wetting agent or a wetting agent formulation, optionally adjusting the impregnated flat-shaped structure to a predetermined wetting agent uptake and optionally drying the material, wherein sodium N-oleoyl-sarcosinate is used as the wetting agent.

10. Process as claimed in claim 9, wherein the applied amount of sodium N-oleoyl-sarcosinate is such that a coating of 0.01 to 2.0 % by weight, preferably 0.03 to 0.5 % by weight of the applied compound relative to the weight of the material before impregnation remains on the porous material.

11. Test strip composed of a flexible flat-shaped support that is optionally transparent or provided with inspection openings, on which one or several test fields are arranged next to one another which each carry one or several detection layers stacked on top of one another, wherein the test fields are covered by an overlay made of a spreading material as claimed in at least one of the claims 1 to 5.

12. Test strip as claimed in claim 11, wherein the spreading overlay is composed of one or several flat-shaped overlay elements which are attached to the test strip in such a way that a part of their surface can be displaced freely relative to the strip surface covered by this part in the direction of curvature produced when the object is bent.

13. Test strip as claimed in claim 12, wherein the test fields are covered by the displaceable zones of an overlay composed of two elements.

14. Test strip as claimed in at least one of the claims 12 and 13, wherein the spreading overlay is composed of two overlay elements whose displaceable regions face one another and overlap.

15. Test strip as claimed in at least one of the claims 11 to 14, wherein the overlap of the two overlay elements is above the separation line between the two test fields and preferably symmetrical thereto.

16. Test strip as claimed in at least one of the claims 11 to 15, wherein it has two single or multilayer test fields for the same or different diagnostically usable analytes which directly adjoin one another or are separated by a gap.

17. Test strip as claimed in at least one of the claims 11 to 16, wherein the arrangement of detection layers and overlays on the test strip is covered with an inert flat-shaped material in such a manner that a space that is adequate for sample application only remains free in the overlap region of the overlay elements viewed in the direction of the longitudinal axis of the test strip.

18. Test strip as claimed in at least one of the claims 11 to 17, wherein the hydrophilicity, transparency and the liquid conducting capacity of the overlay material are matched in such a manner that an analyte sample is distributed over the entire analyte-sensitive region of the test carrier, the test strip is self-dosing and excess sample remains above the application spot.

19. Test strip as claimed in claim 11, wherein it contains one test field which supports a monofilament spreading material as claimed in claims 1 and 3 which is larger than the test field and is attached to the support on both sides of the test field preferably by means of a spacer having the thickness of the test field whereby the part of the spreading material which extends beyond the test field is covered by sample-impermeable material so that a sample application is only possible on that part of the spreading material which rests on the test field.

20. Process for the production of a diagnostic test strip in which single or multilayer detection fields are mounted on a support which is optionally transparent or provided with inspection openings, these are covered with a functional overlay which rests loosely on the test fields and is attached at the sides of the test fields and optionally an inert cover which only leaves the sample application site free is additionally mounted, wherein a functional overlay is used which is composed of a textile material having the features as claimed in one of the claims 1 to 5.

21. Process as claimed in claim 20, wherein a flat-shaped overlay element or several overlapping flat-shaped overlay elements that together form the overlay are attached to the test strip next to the test field(s) in such a way that a part of their surface covers the test field(s) and can move freely relative to the test field(s) in the direction of curvature produced when the object is bent.

22. Use of a diagnostic test strip as claimed in one of the claims 11 to 19 to determine an analyte in a liquid.

23. Method for the determination of an analyte in a liquid sample, in which a sample liquid is applied to the sample application site and the detection layer(s) is(are) observed for signal generation, the signal generation being a measure for the presence or the amount of analyte in the examined liquid sample, wherein a diagnostic test strip as claimed in one of the claims 11 to 19 is used.

**Revendications**

1. Matériau de diffusion, comprenant une structure plane poreuse, imprégnée d'un agent mouillant, **caractérisé en ce que** l'agent mouillant est le N-oléoylsarcosinate de sodium.

2. Matériau de diffusion selon la revendication 1, **caractérisé en ce que** le matériau de diffusion contient 0,01 à 2,0% en poids, de préférence 0,03 à 0,5% en poids, de N-oléoylsarcosinate de sodium, par rapport au poids du matériau avant imprégnation.

3. Matériau de diffusion selon au moins l'une quelconque des revendications 1 à 2, **caractérisé en ce que** la structure plane poreuse à la base du matériau de diffusion selon l'invention est une structure plane textile en monofilaments ou en des fils en multifilaments correspondants.

4. Matériau de diffusion selon au moins l'une quelconque des revendications 1 à 3, **caractérisé en ce que** la structure plane textile est un tissu, un tricot ou un non-tissé, présentant un poids surfacique de 10 à 200, en particulier de 10 à 50 g/m².

5. Matériau de diffusion selon au moins l'une quelconque des revendications 1 à 4, **caractérisé en ce que** le matériau textile présente une épaisseur de 20 à 200, en particulier de 30 à 100 µm et/ou un volume de pores de 30 à 85, en particulier de 40 à 75%.

6. Utilisation de N-oléoylsarcosinate de sodium pour la fabrication d'un matériau de diffusion.

7. Utilisation selon la revendication 6, **caractérisée en ce que** le N-oléoylsarcosinate de sodium est utilisé sous forme pure ou sous forme de solutions ou de préparations liquides.

8. Utilisation selon au moins l'une quelconque des revendications 6 et 7, **caractérisée en ce que** la préparation utilisée contient, outre le N-oléoylsarcosinate de sodium, d'autres adjuvants et/ou additifs.

9. Procédé pour la fabrication d'un matériau de diffusion par imprégnation d'une structure plane poreuse avec un agent mouillant ou une composition d'agent mouillant, le cas échéant réglage de la structure plane imprégnée à une quantité absorbée prédéfinie d'agent mouillant et, le cas échéant, séchage du matériau, **caractérisé ce qu'**on utilise comme agent mouillant du N-oléoylsarcosinate de sodium.

10. Procédé selon la revendication 9, **caractérisé en ce que** la quantité appliquée de N-oléoylsarcosinate de sodium est calculée de telle manière qu'il reste sur le matériau poreux un revêtement de 0,01 à 2,0% en poids, de préférence de 0,03 à 0,5% en poids du composé appliqué, par rapport au poids du matériau avant l'imprégnation.

11. Bandelette test constituée par un support souple, plat, le cas échéant transparent ou pourvu d'ouvertures d'inspection, sur lequel sont disposés un ou plusieurs champs test adjacents, qui portent à chaque fois une ou plusieurs couches de détection superposées, **caractérisée en ce que** les champs test sont recouverts par un revêtement en un matériau de diffusion selon au moins l'une quelconque des revendications 1 à 5.

12. Bandelette test selon la revendication 11, **caractérisée en ce que** le revêtement de diffusion est constitué par un ou plusieurs éléments de revêtement plats, qui sont fixés sur la bandelette test de telle manière qu'une partie de leur surface peut être déplacée librement par rapport à la surface de la bandelette revêtue par cette partie dans le sens d'une courbure obtenue lors de la flexion de l'objet.

13. Bandelette test selon la revendication 12, **caractérisée en ce que** les champs test sont recouverts par les zones planes mobiles d'un revêtement constitué par deux éléments.

14. Bandelette test selon au moins l'une quelconque des revendications 12 et 13, **caractérisée en ce que** le revêtement de diffusion est constitué par deux éléments de revêtement, dont les zones mobiles sont orientées l'une contre l'autre et se recouvrent.

15. Bandelette test selon au moins l'une quelconque des revendications 11 à 14, **caractérisée en ce que** le recouvrement des deux éléments de revêtement se situe au-dessus de la ligne de séparation entre les deux champs test et de préférence symétriquement par rapport à celle-ci.

**16.** Bandelette test selon au moins l'une quelconque des revendications 11 à 15, **caractérisée en ce qu'**elle présente deux champs test à une ou plusieurs couches, directement l'un contre l'autre ou séparés par une fente pour des analytes identiques ou différents, utilisables en diagnostic.

**17.** Bandelette test selon au moins l'une quelconque des revendications 11 à 16, **caractérisée en ce que** la disposition de couches de détection et de revêtements sur la bandelette test est recouverte avec un matériau inerte plat de telle manière qu'il ne reste une zone suffisante pour l'application d'un échantillon que dans la zone du recouvrement des éléments de revêtement, dans le sens de l'axe longitudinal de la bandelette test.

**18.** Bandelette test selon au moins l'une quelconque des revendications 11 à 17, **caractérisée en ce que** l'hydrophilie, la perméabilité et le pouvoir de guidage d'un liquide du matériau de revêtement sont adaptés de telle manière qu'un échantillon avec des analytes est réparti sur toute la zone sensible aux analytes du support test, la bandelette test est auto-dosante, resp. la quantité d'échantillon en excès reste au-dessus du point d'application.

**19.** Bandelette test selon la revendication 11, **caractérisée en ce qu'**elle contient un champ test, sur lequel se trouve un matériau de diffusion monofil selon les revendications 1 et 3, qui est plus grand que le champ test et qui est fixé des deux côtés du champ test sur le support, de préférence via un écarteur, dans l'épaisseur du champ test, la partie du matériau de diffusion qui dépasse du champ test étant revêtue par un matériau imperméable à l'échantillon, de telle manière qu'une application d'échantillon n'est possible que sur la partie du matériau de diffusion qui se trouve sur le champ test.

**20.** Procédé de fabrication d'une bandelette test diagnostique, dans lequel on applique sur un support le cas échéant transparent ou pourvu d'ouvertures d'inspection, des champs de détection à une ou plusieurs couches, on recouvre ceux-ci par un revêtement fonctionnel disposé librement sur les champs, fixé latéralement par rapport aux champs test et on applique le cas échéant encore un recouvrement inerte, qui ne laisse libre que le site d'application de l'échantillon, **caractérisé en ce qu'**on utilise un revêtement fonctionnel qui est constitué par un matériau textile présentant les caractéristiques indiquées dans l'une quelconque des revendications 1 à 5.

**21.** Procédé selon la revendication 20, **caractérisé en ce qu'**on fixe un élément de revêtement plat ou plusieurs éléments de revêtement plats, qui forment ensemble, en se recouvrant, le revêtement, sur la bandelette test à côté du (des) champs test, de telle manière qu'une partie de leur surface recouvre le(s) champs test et peut être déplacée librement par rapport au(x) champ(s) test dans le sens de la courbure obtenue lors de la flexion de l'objet.

**22.** Utilisation d'une bandelette test diagnostique selon l'une quelconque des revendications 11 à 19 pour la détermination d'un analyte dans un liquide.

**23.** Procédé pour la détermination d'un analyte dans un échantillon liquide, dans lequel le liquide de l'échantillon est appliqué sur le site d'application d'un échantillon et on observe la formation d'un signal sur la (les) couches de détection, la formation du signal étant une mesure de la présence resp. de la quantité d'analyte dans l'échantillon liquide analysé, **caractérisé en ce qu'**on utilise une bandelette test diagnostique selon l'une quelconque des revendications 11 à 19.

Fig.1

Fig.2

Fig.3

1

12 11 9 11a 10 9a

6a 6 A'

5a

4a

3a

3 2

5 4 Fig. 4

A 13

10

7 8 8a 7a

9 6 6a 9a Fig. 5

13 2 4 5 3 3a 4a 5a

Fig. 6

2

13 14 15